# EUROPEAN PATENT APPLICATION

(11) **EP 4 785 863 A1**
(43) Date of publication of application: **05.08.2026**
(21) Application number: 26155805.0
(22) Date of filing: 02.02.2026
(51) Int. Cl.: A61B 5/06, A61B 5/287, A61B 5/367

(54) **DEVICE, SYSTEM, AND METHOD FOR ARRHYTHMIA MAPPING WITH MULTI-ELECTRODE MAPPING CATHETER SYSTEMS**

(30) Priority: 03.02.2025 US 202519044304
(71) Applicant: Biosense Webster (Israel) Ltd., 2066717 Yokneam (IL)
(72) Inventor: PARIKH, Paras, Irvine, 92618 (US); BEECKLER, Christopher Thomas, Irvine, 92618 (US); GLINER, Vadim, Irvine, 92618 (US)
(74) Representative: Carpmaels & Ransford LLP

(57) **Abstract**

Methods and systems of mapping electrophysiological signals in a heart using a catheter having an ultra-high density electrode array are disclosed. The disclosed technology can include a method including navigating a medical probe to a target location in a patient's heart; receiving electrophysiological signals from at least some of the electrodes of the plurality of electrodes; identifying, based on the electrophysiological signals and the location signal, an earliest activation point identified as having the earliest activation time; identifying, based on the electrophysiological signals and the location signal, a plurality of points closest to the earliest activation point having an activation time less than a predetermined time duration from the earliest activation time; and generating an electro-anatomical map of the heart based on data corresponding to the plurality of points, the electro-anatomical map representing the location of the earliest activation point for subsequent ablation.

## Description

### CROSS-REFERENCE TO RELATED APPLICATIONS

This application is related to U.S. Patent Application Serial No. 16/723,971 filed 20 December 2019 (Attorney Docket No. BIO6160USNP1), U.S. Patent Application Serial No. 17/489,895 filed 30 September 2021 (Attorney Docket No. BIO6160USCIP1 (253757.000100)), U.S. Patent Application Serial No. 18/159,288 filed 25 January 2023 (Attorney Docket No. BIO6160USCIP2 (253757.000343)), and U.S. Patent Application Serial No. 18/969,596 filed 5 December 2024 (Attorney Docket No. BIO6944USNP1 (253757.000561)), the entire contents and substance of each of which is incorporated herein by reference in its entirety as if fully set forth below.

### FIELD OF THE INVENTION

This disclosure relates to devices and methods of mapping cardiac structures and identifying a location for ablating cardiac tissue using a catheter having a high density of electrodes.

### BACKGROUND

The advancement in high-density diagnostic catheters and mapping systems has contributed to substantially more efficient procedures and improved ablation outcomes compared with conventional systems. In the ablation of complex arrhythmias, high-density mapping has enhanced the visualization of regions of slow conduction (critical isthmus) or macro re-entrant circuits. In addition, rapid electrogram collection with higher density and improved resolution has given rise to shorter mapping and procedure times. In atrial fibrillation ablation procedures, high-density mapping has the potential to visualize previously concealed gaps or areas of dormant conduction, thereby improving pulmonary vein isolation (PVI) durability and reducing the need for re-ablation.

In practice, however, current high-density mapping systems are associated with certain limitations. Interference from far-field signals or noise can result in poor signal fidelity. In complex atrial substrates with multiple atrial potential components, unipolar electrograms referenced to the Wilson Central Terminal are often timed to the wrong component due to large, steep far-field potentials; this can require manual review and corrections of the annotations. Other limitations include electrode spacing and sizing affecting the integrability of ablation and intracardiac echocardiography.

Current multi-electrode catheters having lower numbers of electrodes, ring electrodes, or fixed shape catheters are capable of identifying potential target sites; however, increasing the number and type of electrodes in a globe-shaped, high-density catheter can allow for reduced mapping and procedure time in a clinical setting. The systems and methods of this disclosure are intended to improve the resolution and clarity of electrical signal mapping for electrophysiology procedures.

### SUMMARY

The disclosed technology includes a method comprising navigating a medical probe to a target location in a patient's heart. The medical probe can extend along a longitudinal axis and comprise a plurality of spines configured to bow radially outward from the longitudinal axis. The plurality of spines can comprise a plurality of electrodes disposed thereon and at least a location sensor disposed on the longitudinal axis. The location sensor can be configured to provide a location signal representative of a location of the sensor and the medical probe in the heart.

The method can further include receiving electrophysiological signals from at least some of the electrodes of the plurality of electrodes and identifying, based on the electrophysiological signals and the location signal, an earliest activation point identified as having the earliest activation time. The method can further include identifying, based on the electrophysiological signals and the location signal, a plurality of points closest to the earliest activation point having an activation time less than a predetermined time duration from the earliest activation time. The method can include generating an electro-anatomical map of the heart based on data corresponding to the plurality of points. The electro-anatomical map can represent the location of the earliest activation point for subsequent ablation.

The disclosed technology can further include a medical system comprising a medical probe. The medical probe can comprise a shaft extending along a longitudinal axis, a plurality of spines disposed at a distal end of the shaft and configured to bow radially outward from the longitudinal axis to define a cavity therebetween, and a location sensor disposed on the longitudinal axis. The location sensor can be configured to provide a location signal representative of a location of the sensor and the medical probe in a heart. The medical probe can further include a plurality of electrodes disposed along the plurality of spines and a reference electrode disposed in the cavity.

The medical system can further include one or more processors and a memory storing instructions that, when executed by the one or more processors, are configured to cause the medical system to receive electrophysiological signals from at least some of the electrodes of the plurality of electrodes. The instructions can further cause medical system to identify, based on the electrophysiological signals and the location signal, an earliest activation point identified as having the earliest activation time; identify, based on the electrophysiological signals and the location signal, a plurality of points closest to the earliest activation point having an activation time less than a predetermined time duration from the earliest activation time; and generate an electro-anatomical map of the heart based on data corresponding to the plurality of points. The electro-anatomical map can represent the location of the earliest activation point for subsequent ablation.

To the accomplishment of the foregoing and related ends, certain illustrative aspects are described herein in connection with the following description and the appended drawings. These aspects are indicative, however, of but a few of the various ways in which the principles of the claimed subject matter may be employed and the claimed subject matter is intended to include all such aspects and their equivalents. Other advantages and novel features may become apparent from the following detailed description when considered in conjunction with the drawings.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.

This patent or application file contains at least one drawing executed in color. Copies of this patent or patent application publication with color drawings will be provided by the Office upon request and payment of the necessary fee.
FIG. 1A illustrates a catheter-based electrophysiology mapping and ablation system, according to examples of the presently disclosed subject matter;
FIG. 1B is a perspective view of the basket catheter of FIG. 1A constructed and operative in accordance with an example of the present invention;
FIGs. 2 and 3 are more detailed views of the expandable assembly of the basket catheter of FIGs. 1A and 1B;
FIG. 4 is a partly exploded view of the basket catheter of FIGs. 1A and 1B;
FIG. 5 is an enlarged view of a nose section of the basket catheter of FIGs. 1A and 1B with a nose cap removed;
FIGs. 6A and 6B are schematic views of the expandable assembly of the basket catheter of FIGs. 1A and 1B in expanded and collapsed form;
FIG. 7 is a schematic view of the flexible polymer circuit strips for use in the basket catheter of FIGs. 1A and 1B;
FIG. 8A is a cross-sectional view through line A-A of FIG. 7;
FIGs. 8B-8I illustrate example apertures formed in a covering of the flexible polymer circuit strips of FIG. 7;
FIG. 8J is a table illustrating impedance values for example aperture patterns formed in the covering of the flexible polymer circuit strips of FIG. 7;
FIG. 9 is a schematic view of a deflectable element of the basket catheter of FIGs. 1A and 1B;
FIG. 10 is a schematic view of an irrigation sleeve of the basket catheter of FIGs. 1A and 1B;
FIG. 11 is a schematic view of a pusher of the basket catheter of FIGs. 1A and 1B;
FIG. 12 is a schematic view of a multi-axis position sensor of the basket catheter of FIGs. 1A and 1B;
FIGs. 13A-13B are schematic views of a nose connector of the basket catheter of FIGs. 1A and 1B;
FIG. 14 is a schematic view of a nose connector retainer of the basket catheter of FIGs. 1A and 1B;
FIGs. 15A-15B are schematic views of a nose cap of the basket catheter of FIGs. 1A and 1B;
FIG. 16 is a schematic view of a coupler of the basket catheter of FIGs. 1A and 1B;
FIG. 17 is a schematic view of a single-axis position sensor of the basket catheter of FIGs. 1A and 1B;
FIG. 18 is a schematic view of a proximal retainer ring of the basket catheter of FIGs. 1A and 1B;
FIGs. 19-20 are cross sectional views through line A-A of FIG. 1B;
FIG. 21 illustrates another view of the basket catheter with annotations illustrating various features of the catheter, in accordance with an example of the present invention;
FIG. 22 illustrates views of the basket catheter as rendered on a display showing the basket in an expanded and in a collapsed state, in accordance with an example of the present invention;
FIGs. 23A and 23B illustrate inclusion and exclusion criteria for patients as part of a study of the catheter, in accordance with an example of the present invention;
FIG. 24 is a table illustrating the baseline demographics and comorbidities of participants enrolled in the study, in accordance with an example of the present invention;
FIGs. 25A and 25B is a table of history of atrial and ventricular arrhythmias and ablation procedures of participants enrolled in the study, in accordance with an example of the present invention;
FIG. 26 is a table of procedural characteristics of the procedures completed as part of the study, in accordance with an example of the present invention;
FIGs. 27A-27D represent a graphical representation of a left atrial atypical flutter timing (LAT) and voltage (bipolar) maps of a patient's heart using the catheter, in accordance with an example of the present invention;
FIGs. 28A and 28B represent the intracardiac electrocardiograms recordings observed by the catheter of a patient's heart, in accordance with an example of the present invention;
FIG. 29 illustrates a graphical display of the catheter positioned in a patient's heart to complete a mapping procedure, in accordance with an example of the present invention;
FIG. 30 illustrates a graphical display of the catheter positioned in a patient's heart showing the QS Pre-P wave focal atrial tachycardia site being mapped and identified at the anterior left carina area, in accordance with an example of the present invention;
FIG. 31 is a table summarizing areas of interest identified using the catheter in participants with atrial tachycardia to include PVI triggers, PVI breakthroughs, non-PV AF Foci, left atrial critical isthmus, and Cavo tricuspid isthmus, in accordance with an example of the present invention;
FIG. 32 illustrates a PVC map with the earliest QS pre-QRS site identified at the septal RVOT using the catheter, in accordance with an example of the present invention;
FIG. 33 illustrates an area and perimeter of the earliest activation timing locations calculated and compared to a map's reference, in accordance with an example of the present invention;
FIG. 34 is a table summarizing all PVC cases identifying earliest pot from mid QRS (reference), earliest point pre-QRS, area of the earliest activation points, and perimeter of the earliest activation points, in accordance with an example of the present invention;
FIG. 35 illustrates a flow chart of a method of using the catheter, in accordance with an example of the present invention; and
FIG. 36 illustrates a schematic of a method of using the catheter, in accordance with an example of the present invention.

### DETAILED DESCRIPTION

The disclosed technology includes a catheter having a plurality of spines and a high density of electrodes disposed along the spines. As will become apparent throughout this disclosure, the disclosed system and method can be used to create a more accurate electro-anatomical map of a patient's heart. Further, the disclosed technology can help to accurately identify a location within a heart to be ablated to reduce or eliminate aberrant electrical signals in the heart. In particular, the disclosed technology includes identifying a location of an earliest activation time in the heart and a plurality of points closest to the earliest activation point having an activation time less than a predetermined time duration (e.g., less than one second, less than 10 milliseconds, less than 1 millisecond) from the earliest activation time. In this way, an electro-anatomical map of the patient's heart can be generated identifying a location to be ablated.

Although example embodiments of the disclosed technology are explained in detail herein, it is to be understood that other embodiments are contemplated. Accordingly, it is not intended that the disclosed technology be limited in its scope to the details of construction and arrangement of components set forth in the following description or illustrated in the drawings. The disclosed technology is capable of other embodiments and of being practiced or carried out in various ways.

It must also be noted that, as used in the specification and the appended claims, the singular forms "a," "an" and "the" include plural referents unless the context clearly dictates otherwise. By "comprising" or "containing" or "including" it is meant that at least the named compound, element, particle, or method step is present in the composition or article or method, but does not exclude the presence of other compounds, materials, particles, method steps, even if the other such compounds, material, particles, method steps have the same function as what is named.

As used herein, the terms "about" or "approximately" for any numerical values or ranges indicate a suitable dimensional tolerance that allows the part or collection of components to function for its intended purpose as described herein. More specifically, "about" or "approximately" can refer to the range of values ±20% of the recited value, e.g. "about 90%" can refer to the range of values from 71% to 99%.

As discussed herein, a heart or vasculature of a "subject" or "patient" can be a heart or vasculature of a human or any animal. The term "proximal" means that an object is closer to the physician and distal means that the object is further away from the physician.

As used herein in the context of a circuit strip, the term 'longitudinal' refers to the direction along the length of the strip, extending from the proximal end to the distal end. The term 'lateral' refers to the direction perpendicular to the longitudinal axis, spanning the width of the strip. The term 'thickness' refers to the dimension perpendicular to both the longitudinal and lateral directions, indicating the depth of the strip from the top surface (i.e. top layer) to the bottom surface (bottom layer).

In the following detailed description, numerous specific details are set forth in order to provide a thorough understanding. However, it will be understood by those skilled in the art that the presently disclosed subject matter may be practiced without these specific details. In other instances, well-known methods and features have not been described in detail so as not to obscure the presently disclosed subject matter.

In describing example embodiments, terminology will be resorted to for the sake of clarity. It is intended that each term contemplates its broadest meaning as understood by those skilled in the art and includes all technical equivalents that operate in a similar manner to accomplish a similar purpose. It is also to be understood that the mention of one or more steps of a method does not preclude the presence of additional method steps or intervening method steps between those steps expressly identified. Steps of a method may be performed in a different order than those described herein without departing from the scope of the disclosed technology. Similarly, it is also to be understood that the mention of one or more components in a device or system does not preclude the presence of additional components or intervening components between those components expressly identified.

### Description of System

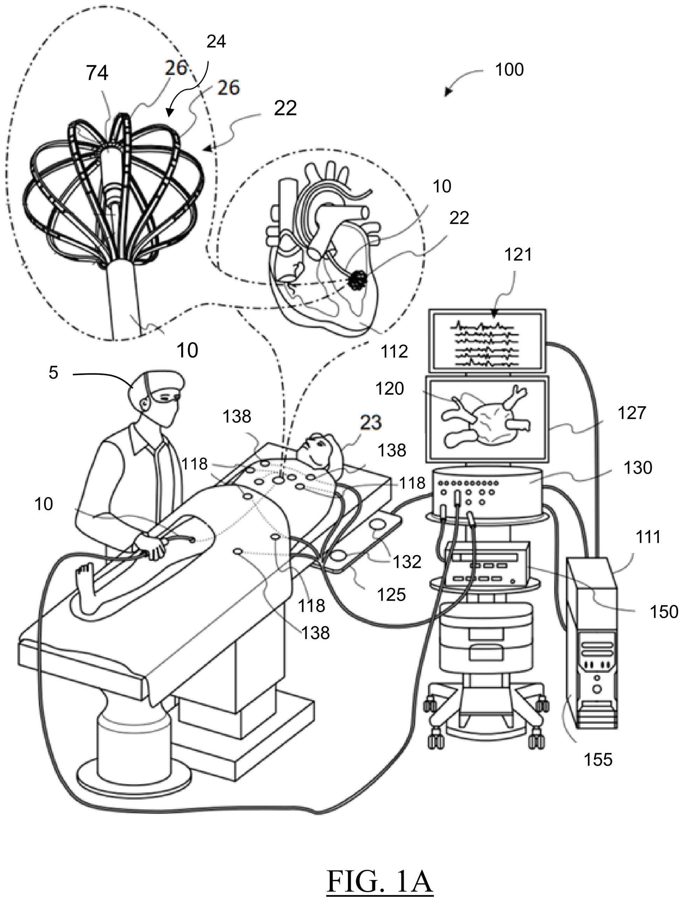

Reference is made to FIG. 1A showing an example catheter-based electrophysiology mapping and ablation system 100. System 100 includes multiple catheters, which are percutaneously inserted by a physician 5 through the patient's vascular system into a chamber or vascular structure of a heart 112. Typically, a delivery sheath catheter is inserted into the left or right atrium near a desired location in heart 112. Thereafter, one or more catheters may be inserted into the delivery sheath catheter so as to arrive at the desired location in heart 112. The plurality of catheters may include catheters dedicated for sensing Intracardiac Electrogram (IEGM) signals, catheters dedicated for ablating and/or catheters dedicated for both sensing and ablating. An example catheter 10 that is configured for ablating tissue and/or for sensing electrical cardiac activity and/or mapping is illustrated in the inset in FIG. 1A.

Catheter 10 is an exemplary catheter having an end effector at its distal tip that includes an expandable assembly 22, having one and preferably multiple electrodes 26 optionally distributed over a plurality of flexible spine elements 24 (sometimes referred to herein as "flexible polymer circuit strips 24"). The electrodes 26 are generally configured for delivering ablation energy to tissue and/or for sensing electrophysiological signals (e.g, IEGM signals). Catheter 10 additionally includes one or more position sensors 74 embedded in or near distal tip for tracking position and orientation of distal tip. Optionally and preferably, position sensor 74 is a magnetic based position sensor, for example a position sensor including three magnetic coils for sensing three-dimensional (3D) position and orientation; or a position sensor including one magnetic coil, for sensing a single direction. In some examples, the catheter 10 can include a first position sensor 74 disposed near a proximal end of the expandable assembly 22 and a second position sensor 74 disposed near a distal end of the expandable assembly. In this way, the positions of the first position sensor 74 and the second position sensor 74 can be used to determine an elongation and shape of the expandable assembly 22. This can be useful for determining when the expandable assembly 22 is ready to be retracted back into a sheath and when the expandable assembly is fully deployed for mapping and/or ablation.

Each of the magnetic based position sensors 74 may be operated together with a location pad 125 including a plurality of magnetic coils 132 configured to generate magnetic fields in a predefined working volume. Real time position of distal tip of catheter 10 may be tracked based on magnetic fields generated with location pad 125 and sensed by magnetic based position sensor 74. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, the entireties of each of which are incorporated herein by reference.

The physician 5 may place a distal tip of catheter 10 in contact with the heart wall for sensing a target site in heart 112. For ablation, the physician 5 may similarly place a distal end of an ablation catheter in contact with a target site for ablating tissue.

System 100 includes one or more electrode patches 138 positioned for skin contact on patient 23 to establish location reference for location pad 125 as well as impedance-based tracking of electrodes 26. For impedance-based tracking, electrical current is directed to electrodes 26 and sensed at electrode skin patches 138 so that the location of each electrode can be triangulated via the electrode patches 138. Details of the impedance-based location tracking technology are described in US Patent Nos. 7,536,218; 7,756,576; 7,848,787; 7,869,865; and 8,456,182, the entireties of each of which are incorporated herein by reference.

A recorder 111 records and displays electrograms 121 captured with body surface ECG electrodes 118 and intracardiac electrograms (IEGM) captured with electrodes 26 of catheter 10. Recorder 111 may include pacing capability for pacing the heart rhythm and/or may be electrically connected to a standalone pacer.

System 100 may include an ablation energy generator 150 that is adapted to conduct ablative energy to one or more of electrodes at a distal tip of a catheter configured for ablating. Energy produced by ablation energy generator 150 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 130 is an interface configured to establish electrical communication between catheters, other electrophysiological equipment, power supply and a workstation 155 for controlling operation of system 100. Electrophysiological equipment of system 100 may include for example, multiple catheters, location pad 125, body surface ECG electrodes 118, electrode patches 138, ablation energy generator 150, and recorder 111. Optionally and preferably, PIU 130 additionally includes processing capability for implementing real-time computations of location of the catheters and for performing ECG calculations.

Workstation 155 includes memory, processor unit with memory or storage with appropriate operating software stored therein, and user interface capability. Workstation 155 may provide multiple functions, optionally including (1) modeling the endocardial anatomy in three-dimensions (3D) and rendering the model or anatomical map 120 for display on a display device 127, (2) displaying on display device 127 activation sequences (or other data) compiled from recorded electrograms 121 in representative visual indicia or imagery superimposed on the rendered anatomical map 120, (3) displaying real-time location and orientation of multiple catheters within the heart chamber, and (4) displaying on display device 127 sites of interest such as places where ablation energy has been applied or is to be applied. One commercial product embodying elements of the system 100 is available as the CARTO^{®} 3 System, available from Biosense Webster, Inc., 31 Technology Drive, Suite 200, Irvine, CA 92618.

In some examples, investigational catheter is a multielectrode ECG mapping catheter that works in conjunction with the CARTO^{®} 3 EP Navigation System. It is designed for deployment in a heart chamber through an 8.5 F guiding sheath. In some examples, and as will be described in greater detail herein, this bi-directional deflectable catheter can include ten spines in a basket shape on its deflectable tip, each spine having ten coated electrodes that are used for stimulating and recording. Additionally, the catheter 10 can include two position sensors 74 located along the longitudinal axis near the distal end of the catheter 10.

In some examples, an irrigation module is provided for delivering irrigation fluid, such as saline solution, to the location of treatment. The irrigation module may comprise a pump and an associated fluid tank.

### Description of Catheter

### Overview

The deflectable bidirectional mapping catheter described herein is designed with one hundred electrodes coated over 10 spines to form a basket shape. It has an 8Fr shaft compatible with an 8.5Fr sheath for advancement into the heart chamber. The basket has an adaptable size for the diameter, from a minimum of 3 mm to a maximum of 18 mm, and maps can be acquired throughout the range of deployment depending on the need at a particular anatomical location. The basket array consists of 10 nitinol spines with a laminated flexible printed circuit with 10 tiny outward facing gold electrodes (surface area of 0.5 mm2 and 1.7 mm interelectrode spacing, center-to-center) on each spine (total of 100 electrodes), connected proximally to a flat-nose tip. The electrodes are flat with an impedance-reducing coating to allow for decreased electrical impedance, improved signal quality, and superior signal to noise ratio compared with previous high-density mapping catheters.

Three magnetic sensors, 2 in the distal portion and 1 in the proximal portion, are embedded in the basket array that transmit location and angle information independent of advanced catheter location to the CARTO^{®} 3 System (Biosense Webster, Inc.) with a system accuracy of <1 mm. Below the spines on the deflectable tip are two electrodes that allow visualization of the shaft on the CARTO^{®} 3 System. The TRUEref Electrode (Biosense Webster, Inc.), which is embedded in the center of the globe, can be used as a non-contact central reference electrode or a close unipolar reference electrode within the heart chamber allowing enhanced qualifying and filtering of incoming signals for map annotation. CARTO^{®} 3 software version 7 is compatible to reduce the need of manual annotation; an added feature included an automatic outlier detection and binning of points algorithm, termed "Wisdom of the crowd" (WofC). As will be described in greater detail herein, this feature removes outlier timing or voltage annotation based on a certain criterion collected within a 1-mm³ pre-defined fast anatomical mapping (FAM) area (voxel).

### Detailed Description of the Catheter

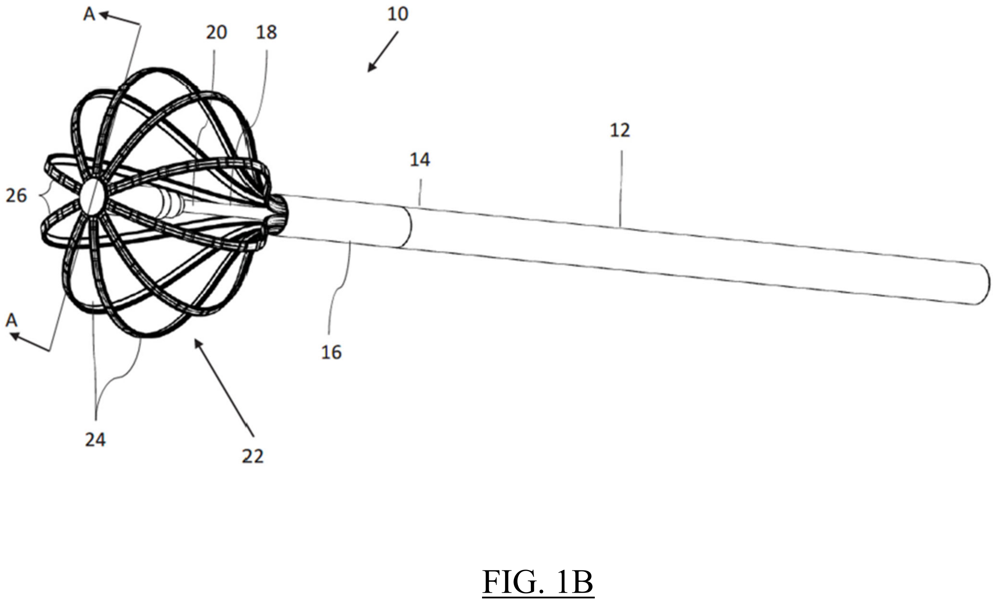

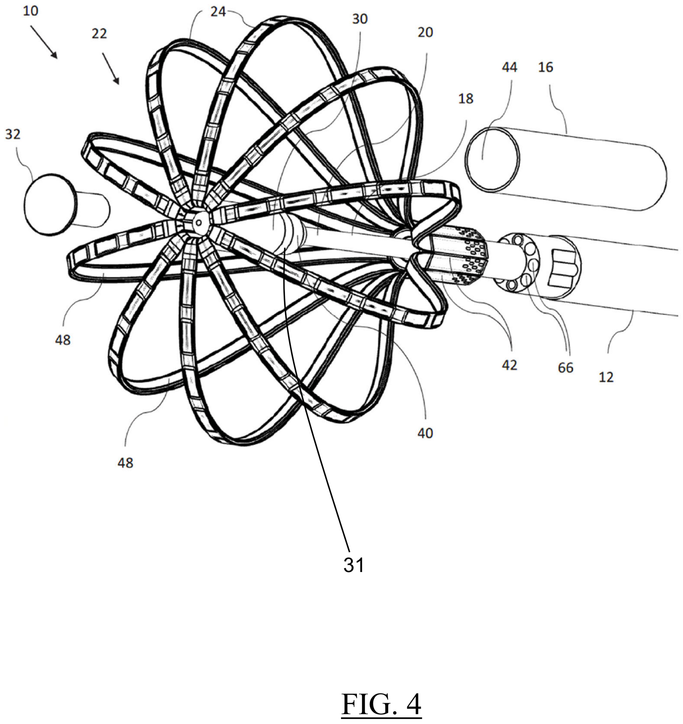

Reference is now made to FIG. 1B, which is a schematic view of a basket catheter 10 constructed and operative in accordance with an embodiment of the present invention. The basket catheter 10 includes an elongated deflectable element 12 having a distal end 14, a coupler 16 connected to the distal end 14, and a pusher 18 including a distal portion 20. The pusher 18 is configured to be advanced and retracted through the deflectable element 12, for example, using a manipulator or handle (not shown). The basket catheter 10 also includes an expandable assembly 22 comprising a plurality of flexible polymer circuit strips 24 (sometimes referred to herein as "spines" - only some have been labeled for the sake of simplicity). Each flexible polymer circuit strip 24 includes multiple electrodes 26 disposed thereon (only some labeled for the sake of simplicity). The formation of the various elements and how they are connected with each other are described in more detail with reference to the FIGs. 4-20.

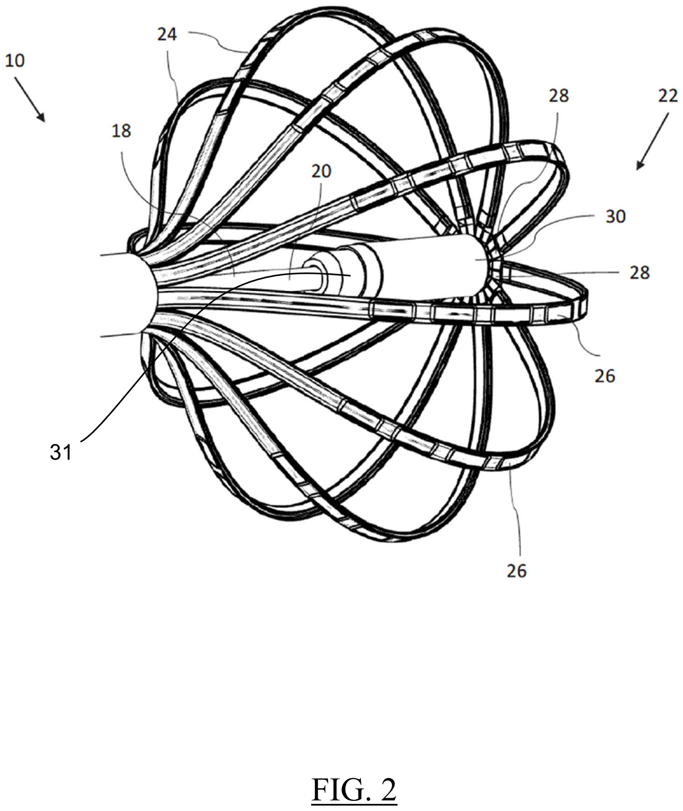

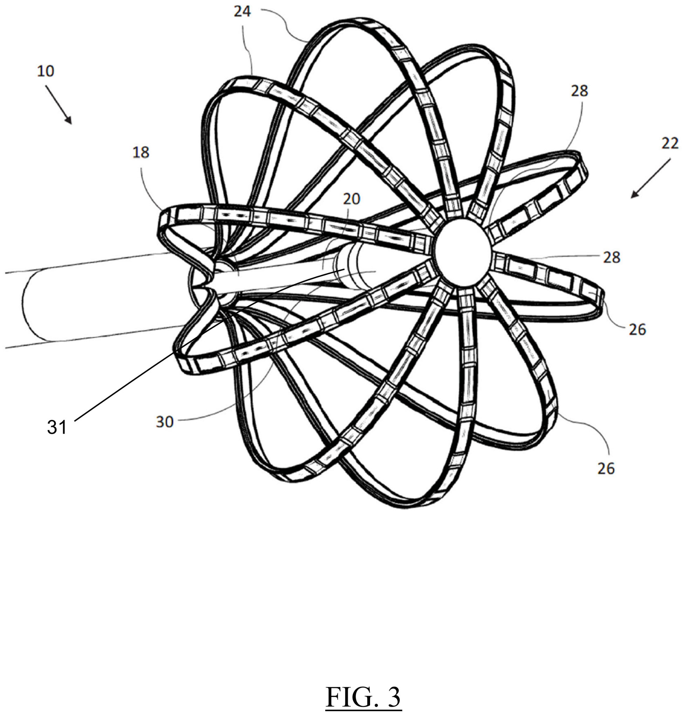

Reference is now made to FIGs. 2 and 3, which are detailed views of the expandable assembly 22 of the basket catheter 10 of FIGs. 1A and 1B. FIGs. 2 and 3 show the electrodes 26 on the flexible polymer circuit strips 24 more clearly. FIG. 2 shows that the electrodes 26 are not disposed on the proximal portions of the flexible polymer circuit strips 24, although they could be in some examples. The basket catheter 10 includes a nose connector 30 connected to the distal portion 20 of the pusher 18. The flexible polymer circuit strips 24 are connected via hinges 28 (only some labeled for the sake of simplicity) of the flexible polymer circuit strips 24 to the nose connector 30.

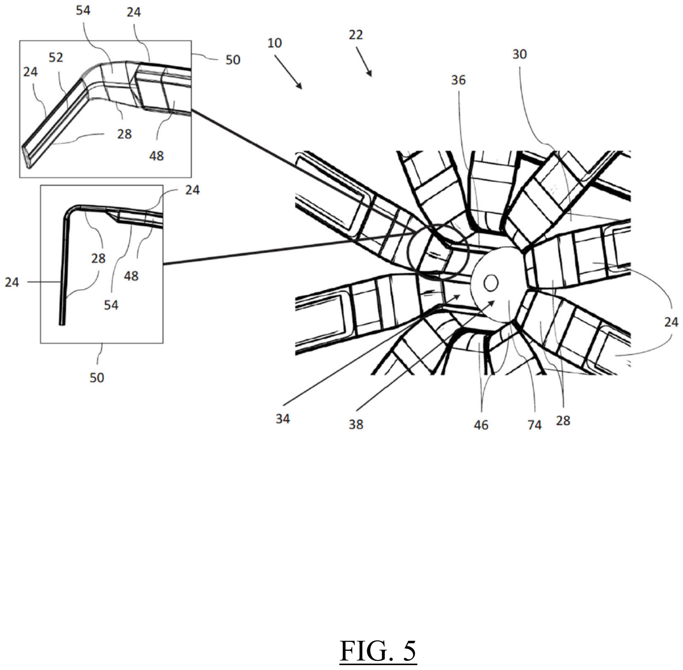

Reference is now made to FIGs. 4-5. FIG. 4 is a partly exploded view of the basket catheter 10 of FIGs. 1A and 1B. FIG. 5 is an enlarged view of a nose section of the basket catheter 10 of FIGs. 1A and 1B with a nose cap 32 removed.

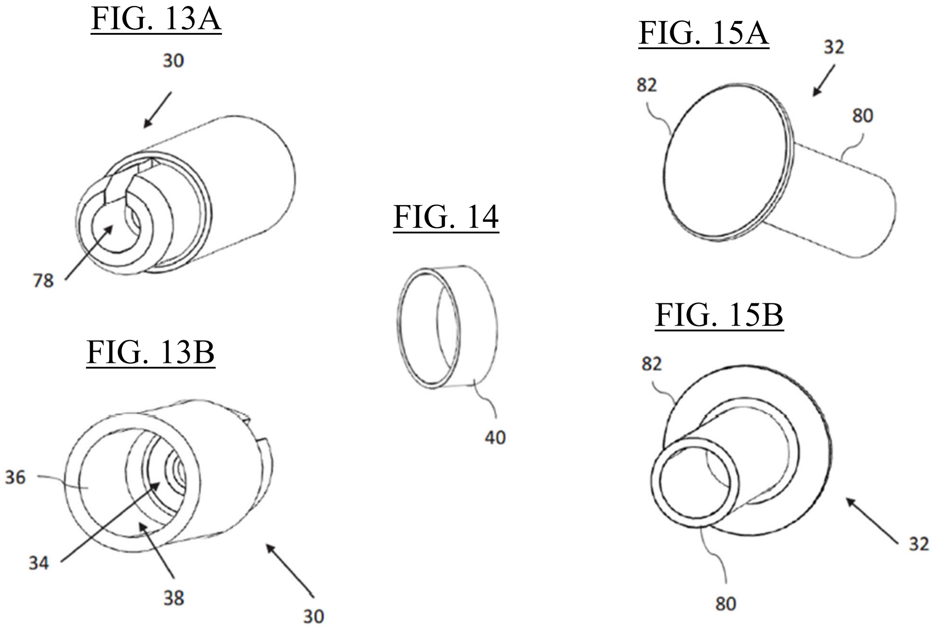

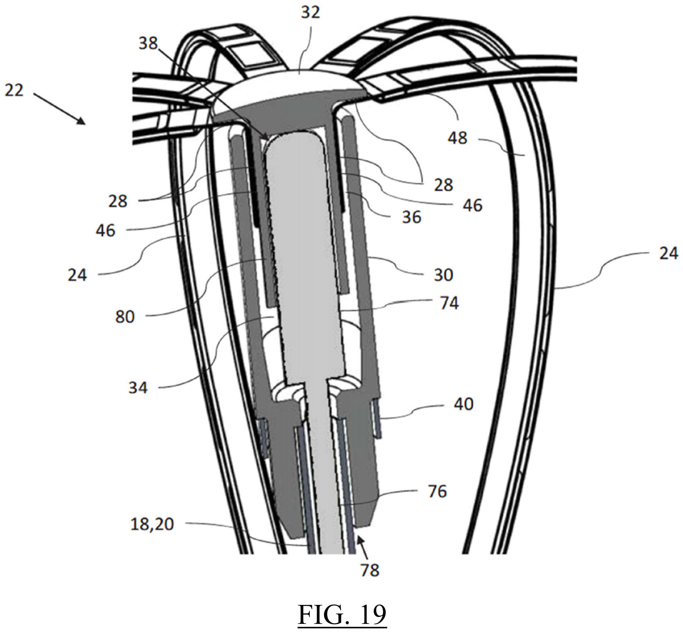

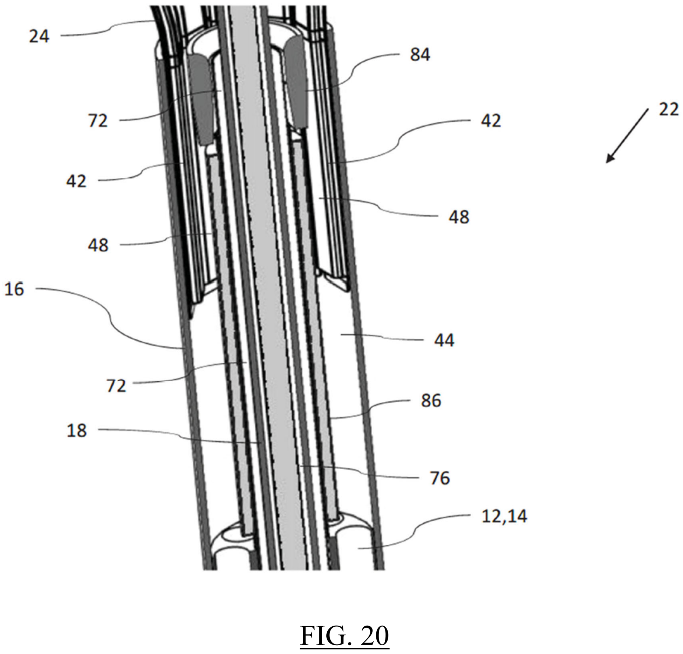

FIG. 4 shows the nose cap 32 and the coupler 16 removed from the basket catheter 10 to illustrate how the flexible polymer circuit strips 24 are connected to the nose connector 30 and the coupler 16. The nose connector 30 is connected to the distal portion 20 of the pusher 18. The proximal end of the coupler 16 may be connected to the elongated deflectable element 12 using any suitable connection method, such as using adhesive, for example, epoxy. The nose connector 30 is secured to the distal portion 20 of the pusher 18 using a center electrode ring 40, which is described in more detail with reference to FIGs. 14 and 19. The flexible polymer circuit strips 24 are disposed circumferentially around the distal portion 20 of the pusher 18, with first ends 42 (only some labeled for the sake of simplicity) of the strips 24 being connected to an inner surface 44 of the coupler 16. The connection between the flexible polymer circuit strips 24 and the inner surface 44 is shown more clearly with reference to FIG. 20.

As shown in FIGs. 2-4, the catheter can further include a reference electrode 31 that can be mounted in a middle of the expandable assembly 22 along a longitudinal axis extending through the center thereof. The reference electrode 31 can be configured to detect electrophysiological signals propagated through blood or other fluid and used to reduce noise detected by the electrodes 26. As will be appreciated, electrophysiological signals detected by the reference electrode 31 can be compared to, or subtracted from, electrophysiological signals detected by electrodes 26 in contact with tissue to reduce or eliminate far field noise to achieve a more accurate reading of the electrophysiological signals propagating through tissue.

The disclosed technology can be configured to measure electrophysiological signals at an electrode 26 on the spines 24, at more than one electrode 26 on the spines, and/or at the reference electrode 31 to acquire accurate electrophysiological signals. For example, a unipole can be measured between an electrode 26 on a spine 24 and the reference electrode 31 to eliminate far field noise. Further, a bipole can be measured between two or more electrodes 26 on the spines 24 to determine electrophysiological signals at each electrode 26 and to determine electrophysiological signals between predetermined electrodes 26. For example, a bipole can be achieved between a first electrode 26 on a spine 24 and a second electrode 26 on the same spine 24. Alternatively, a bipole can be achieved between a first electrode 26 on a first spine 24 and a second electrode 26 on a second spine 24. As will be appreciated, the disclosed technology can be configured to measure and compare electrophysiological signals between any of the electrodes 26 and/or the reference electrode 31 to achieve highly-accurate electrophysiological measurements.

Furthermore, the disclosed technology can be configured correlate location of a particular electrode 26 with the electrophysiological data obtained by the particular electrode 26. In this way, the disclosed technology can be configured to accurately output position and electrophysiological data to generate an high-density electrophysiological map of a patient's heart.

FIG. 5 shows that the nose connector 30 includes a distal receptacle 34 having an inner surface 36 and a distal facing opening 38. The nose connector 30 is described in more detail with reference to FIGs. 13A-B and 19. FIG. 5 shows that second ends 46 (FIG. 5) (only some labeled for the sake of simplicity) of the strips 24 comprising the respective hinges 28 (FIG. 5) entering the distal facing opening 38 (FIG. 5) and are connected to the inner surface 36 (FIG. 5) of the distal receptacle 34 (FIG. 5) of the nose connector 30.

FIG. 4 shows that the basket catheter 10 also includes respective elongated resilient support elements 48 connected along a given length of respective ones of the flexible polymer circuit strips 24 providing a shape of the expandable assembly 22 in the expanded form of the expandable assembly 22. The elongated resilient support elements 48 may form, or be part of, the spines and include any suitable material, for example, but not limited to, Nitinol and/or Polyetherimide (PEI).

FIG. 4 shows that the respective elongated resilient support elements 48 extend along inner surface of the respective strips 24 from the coupler 16, while FIG. 5 shows that the elongated resilient support elements 48 extend along the respective flexible polymer circuit strips 24 until before the respective hinges 28. Insets 50 of FIG. 5 show one of the hinges 28 and a portion of one of the flexible polymer circuit strips 24 adjacent to that hinge 28. The insets 50 illustrate that the elongated resilient support element 48 does not extend to the region of the hinge 28. It can also be seen that the hinge region is much thinner than the region including the elongated resilient support element 48. The hinges 28 may have any suitable thickness, for example, in the range of approximately 10 to approximately 140 microns. The strip 24 are folded such that strip 24 defines a generally perpendicular configuration (inset 50) to each other.

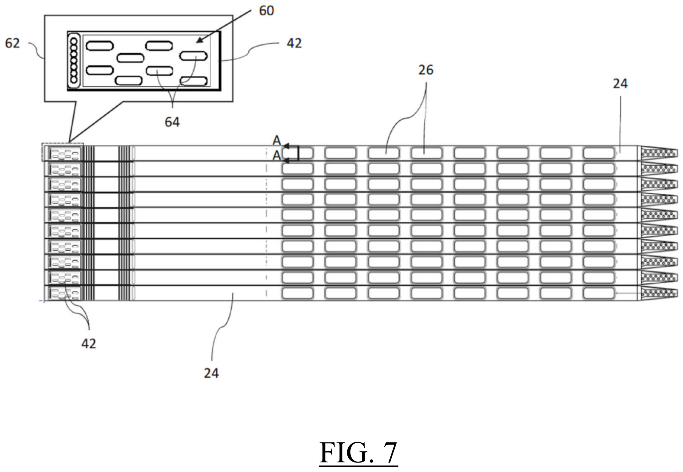

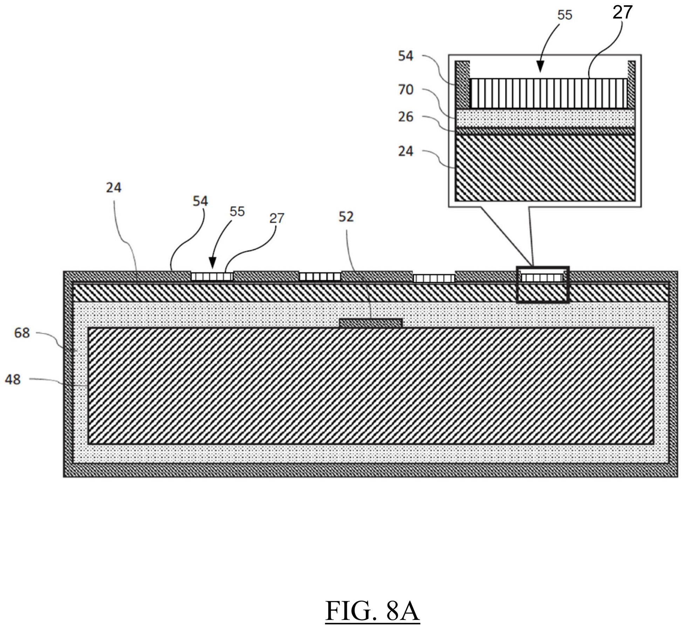

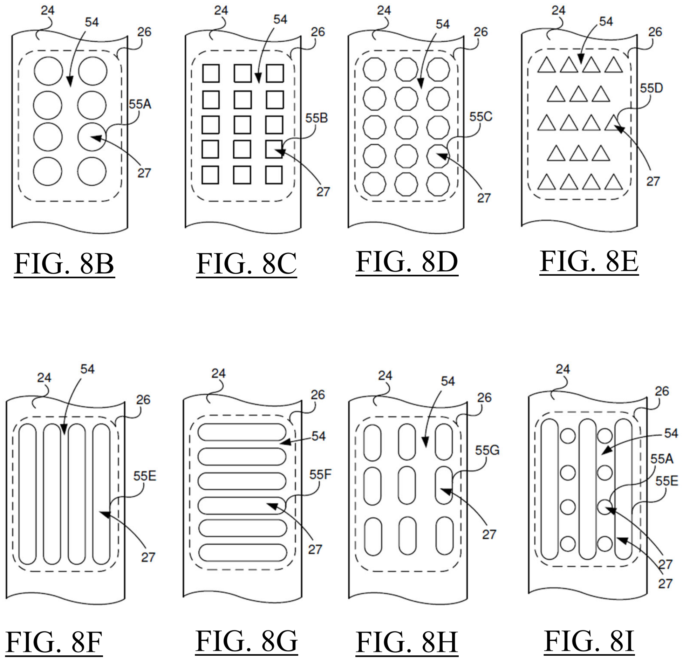

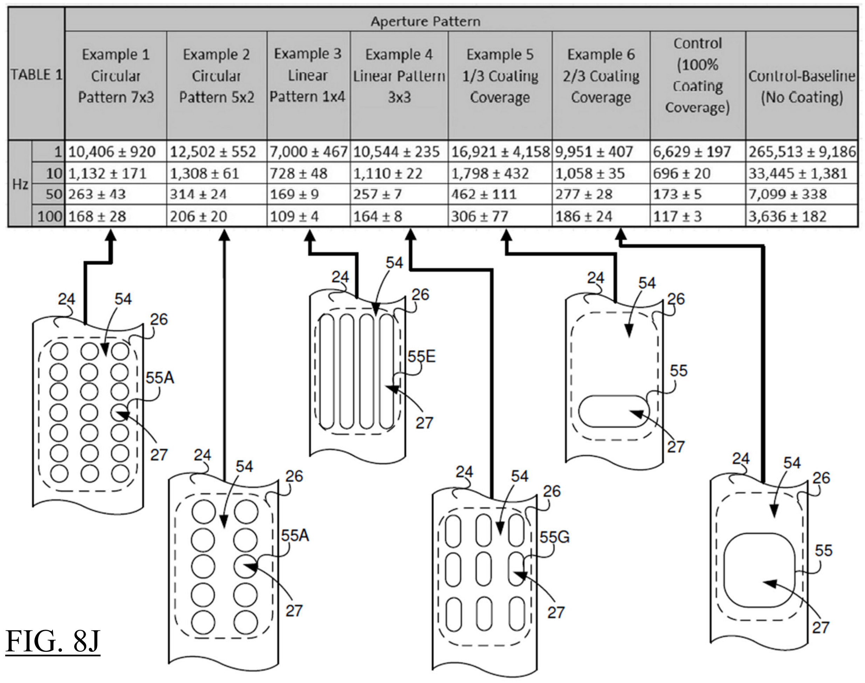

In some embodiments, each of the flexible polymer circuit strips 24 comprises a polyimide layer. The flexible polymer circuit strips 24 may be composed of any suitable materials. The flexible polymer circuit strips 24 are described in more detail with reference to FIGs. 7 and 8.

FIG. 5 also shows that respective ones of the second ends 46 of respective ones of the flexible polymer circuit strips 24 are tapered along the width of the respective ones of the flexible polymer circuit strips 24 to allow inserting the second ends 46 into the distal receptacle 34 without overlap. The hinges 28 may be connected to the inner surface 36 of the distal receptacle 34 using any suitable adhesive, for example, epoxy, and/or using any suitable connection method.

The hinges 28 of the flexible polymer circuit strips 24 are supported with a length of yarn 52, which typically runs the length of each respective flexible polymer circuit strip 24. Each flexible polymer circuit strip 24 along with the yarn 52 and the associated elongated resilient support element 48 may be covered with a suitable covering 54, e.g., thermoplastic polymer resin shrink wrap (PET) described in more detail with reference to FIG. 8A. Yarn 52 can be any suitable high strength polymer including, for example, ultra high molecular weight polyethylene (Spectra or Dyneema), Kevlar, liquid crystal polymer (Vectran) and the like.

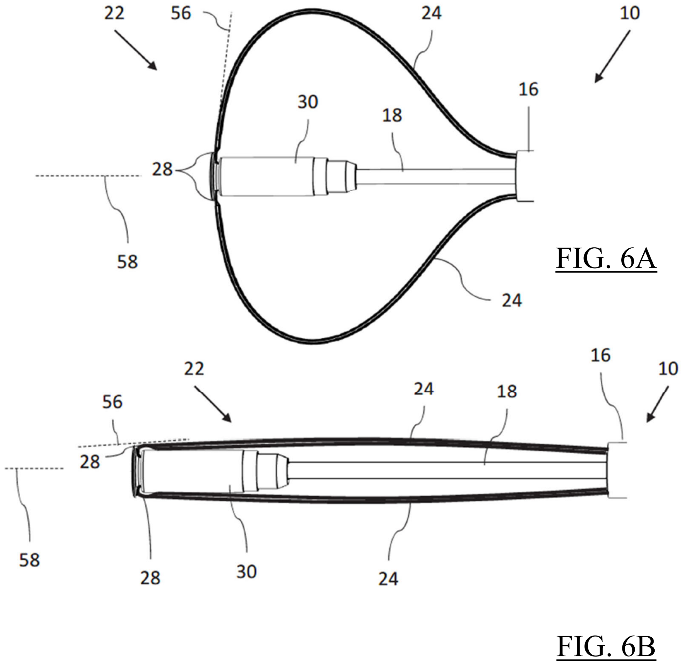

Reference is now made to FIGs. 6A and 6B, which are schematic views of the expandable assembly 22 of the basket catheter 10 of FIGS. 1A and 1B in expanded and collapsed form, respectively. The flexible polymer circuit strips 24 are configured to bow radially outward when the pusher 18 is retracted expanding the expandable assembly 22 from a collapsed form to an expanded form. The collapsed form of the expandable assembly 22 represents the non-stressed form of the flexible polymer circuit strips 24 which are provided with their shape using the elongated resilient support elements 48 (FIG. 4).

In some embodiments, the flexible polymer circuit strips 24 are formed as flat strips as described in more detail with reference to FIG. 7. The distal ends of the flexible polymer circuit strips 24 are connected to the inner surface 36 (FIG. 5) of the nose connector 30. At that point the flat flexible polymer circuit strips 24 are generally parallel with a line 58, which is an extension of an axis of the nose connector 30 extended distally beyond the distal end of the nose connector 30. The proximal ends of the flexible polymer circuit strips 24 are then connected to the coupler 16 so that in the collapsed form, the angle between a tangent 56 to the flexible polymer circuit strips 24 and the line 58 is close to 180 degrees, while in the expanded form, the angle between the tangent 56 and the line 58 is about 90 degrees. Therefore, in operation (when the flexible polymer circuit strips 24 are connected to the nose connector 30 and the coupler 16) the hinges 28 are configured to provide a maximum angular range of movement of the flexible polymer circuit strips 24 of about 90 degrees and generally in excess of 80 degrees. However, the hinges 28 are capable of bending 180 degrees or more. The maximum angular range is defined as the maximum angular range between the tangent 56 to the flexible polymer circuit strips 24 and the line 58. The tangent 56 to the most distal portion of the flexible polymer circuit strips 24 generally provides the maximum angular range between the flexible polymer circuit strips 24 and the line 58.

Reference is now made to FIG. 7, which is a schematic view of the flexible polymer circuit strips 24 for use in the basket catheter 10 of FIGS. 1A and 1B. The flexible polymer circuit strips 24 may be formed from a single piece of polymer, such as polyimide. Circuit strips 24 may be connected to each other by polyimide, or assembled as individual pieces that are held in proper alignment and secured to coupler 16. By manufacturing circuit strips 24 as individual components the yield of the base circuit may be increased as a failed electrode scraps one circuit strip rather than an entire assembly of strips. Respective first ends 42 of the respective flexible polymer circuit strips 24 include an electrical connection array 60. An inset 62 shows that the electrical connection array 60 includes electrical contacts 64 thereon (only some labeled for the sake of simplicity). The electrical contacts 64 are connected via traces (not shown) on the back of the flexible polymer circuit strips 24 to respective ones of the electrodes 26 disposed on the front of the flexible polymer circuit strips 24. Away from the region of the first ends 42, the flexible polymer circuit strips 24 are separate from each other to allow the flexible polymer circuit strips 24 to form the expandable assembly 22 (FIGs. 1A and 1B) when connected to the basket catheter 10. Wires (not shown) may connect the electrodes 26 to control circuitry (not shown) via the electrical contacts 64. The wires may be disposed in lumens 66 (FIG. 4) of the elongated deflectable element 12 (FIG. 4).

The flexible polymer circuit strips 24 may have any suitable dimensions. For example, the length of the flexible polymer circuit strips 24 may be in the range of 10mm to 60mm, e.g., 30 mm the width of the flexible polymer circuit strips 24 may be in the range of 0.25mm to 3mm, e.g., 0.72 mm, the thickness of the flexible polymer circuit strips 24 may be in the range of 0.005 mm to 0.14mm.

Reference is now made to FIG. 8A, which is a cross-sectional view through line A-A of FIG. 7. The yarn 52 is run along the length of the elongated resilient support element 48, e.g., formed from Nitinol or PEI, and beyond so that the yarn 52 will also run the length of the hinge 28 comprised of the flexible polymer circuit strips 24. The elongated resilient support elements 48 may have any suitable thickness, for example, in the range of 0.025 mm to 0.25 mm. A covering 68, such as a thermoplastic polymer resin shrink wrap (PET), is placed over the yarn 52 and the elongated resilient support element 48. Epoxy is injected into the covering 68. Heat is then applied to the covering thereby shrinking the covering over the yarn 52 and the elongated resilient support element 48. One reason to cover the elongated resilient support element 48 with the covering 68 is to electrically isolate the elongated resilient support element 48 from the circuit traces of the flexible polymer circuit strip 24. The covering 68 may be omitted, for example, if the elongated resilient support element 48 is covered with an insulating coating (e.g., polyurethane) or is comprised of an insulating material.

The yarn 52 may comprise any one or more of the following: an ultra-high-molecular-weight polyethylene yarn; or a yarn spun from a liquid-crystal polymer. The yarn 52 may be any suitable linear density, for example, in a range between 25 denier and 250 denier.

The flexible polymer circuit strip 24 are then placed over the yarn 52 and the elongated resilient support element 48 with the circuit trace side of the flexible polymer circuit strip 24 facing the elongated resilient support element 48 and the electrodes 26 of the flexible polymer circuit strips 24 facing away from the elongated resilient support element 48. The covering 54 is disposed around the flexible polymer circuit strip 24, yarn 52, and elongated resilient support element 48 combination, and epoxy 70 is injected into the covering 54. The covering 54 is then heated thereby shrinking the covering 54 around the combination. The flexible polymer circuit strips 24 are therefore covered with the covering 54, e.g., a thermoplastic polymer resin shrink wrap (PET).

As illustrated in FIG. 8A, apertures 55 can be formed through the covering 54 to expose the electrode 26. In some examples, the apertures 55 can expose the entire outer surface of each electrode 26 or the apertures can expose only a portion of the outer surface of each electrode 26. The apertures 55 can be formed by using a laser to cut, or otherwise remove, the covering 54 to expose the electrode 26. In other examples, the apertures 55 can be formed by mechanically removing the covering 54, by chemically etching the covering, plasma etching the covering, or by other suitable methods of removing the covering 54 to form the apertures 55. The covering 54 can be removed such that the conductive surface of each electrode 26 is disposed approximately 12 microns below an outer surface of the covering 54. As will be described in greater detail in relation to FIGs. 8B-8I, if the apertures 55 expose only a portion of the outer surface of each electrode 26, the apertures 55 can comprise several small apertures 55 which collectively define a conductive area that is less than 50% of the conductive surface of the electrode 26.

Some or all of the electrodes 26 can also be coated with a coating 27 to help ensure the electrode 26 is able to properly detect electrical signals of the heart. The coating 27 can be any type of coating suitable for the application. As a non-limiting example, the coating 27 can be poly(3,4-ethylenedioxythiophene) (PEDOT), poly(3, 4 ethylenedioxythiophene) polystyrene sulfonate (PEDOT:PSS), electrochemically grown iridium oxide, electrochemically grown Titanium Nitride (TiN) or any other suitable coating for the particular application. The coating 27 can help to reduce the overall impedance of the electrode 26. In some examples, the coating 27 can be applied to the exposed surface of the electrode 26 such that the overall impedance can be reduced by about 99% at low frequencies. As an example, the coating 27 can be configured such that the input impedance to each electrode 26 is measured at less than 13,000 ohms at 1 Hz.

The coating 27 can be a hydrogel that can be electrochemically grown or adhered to the electrode 26 when a current is passed through the electrode 26. In other examples, the coating 27 can be mechanically applied to each electrode 26 by spraying, painting, dipping, or otherwise covering the electrode 26 with the coating 27. The coating can have a thickness between 10 nanometers and 10 microns. In some examples, the coating can be a thickness that is less than the thickness of the covering 54 such that the covering 54 can help to protect the coating 27 from contacting the coupler 16, the deflectable element 12, or other objects which can damage the coating 27.

Reference is now made to FIGs. 8B-8I, which illustrate example apertures 55 formed in a covering of the flexible polymer circuit strips of FIG. 7. By forming apertures 55 formed through the covering 54 to expose a surface of the electrode 26, the exposed surface of the electrode 26 can be coated with the coating 27. As illustrated in FIGs. 8B-8I, the apertures 55 can be many shapes, sizes, and configurations. As will be appreciated by one of ordinary skill in the art, by changing the shape, size, and configuration of the apertures 55, the amount of exposed surface area of the electrodes 26 can be either increased or decreased, effectively increasing or decreasing the conductive surface area of the electrode 26. Furthermore, by increasing or decreasing the exposed surface area of the electrodes 26, the amount of coating 27 that can be applied to an aperture 55 will also be increased or decreased. In other words, as the apertures 55 increase in size, the surface area of the coating 27 in each aperture 55 will also increase which can cause the coating 27 to be more likely to rub on objects and become delaminated. Thus, as the aperture 55 size decreases, the covering 54 can provide more mechanical protection to the coating 27 to help reduce the likelihood of the coating 27 coming into contact with objects as the basket catheter 10 is used. As will be appreciated, however, as the size of a given aperture 55 is reduced, the conductive surface area of the electrode 26 will also be reduced. Therefore, the size, shape, and configuration of the apertures 55 over an electrode 26 can be optimized to allow for the electrode to sufficiently detect electrical signals while also ensuring the covering 54 provides sufficient mechanical protection to the coating 27. Manufacturability is another consideration. Presently, it is preferred that features of the covering 54 are at least 0.003 inches (76 microns) to avoid damaging the covering 54 between apertures 55 during manufacturing.

FIG. 8B illustrates an example electrode 26 of a flexible polymer circuit strip 24 having circular apertures 55A through the covering 54. In this example, eight circular apertures 55A can be formed through the covering 54 with each circular aperture 55A being spaced equally from each other. As will be appreciated, more or fewer circular aperture 55A can be formed through the covering 54 depending on the application. Furthermore, in some examples, the circular apertures 55A can be unequally spaced from each other. The coating 27 can be adhered to the exposed surface of the electrode 26 within each circular aperture 55A.

In other examples, the apertures 55 can comprise a polygonal shape. For example, FIG. 8C illustrates an electrode 26 of a flexible polymer circuit strip 24 having rectangular apertures 55B through the covering 54. In this example, fifteen rectangular apertures 55B can be formed through the covering 54 with each rectangular aperture 55B being spaced equally from each other. The rectangular apertures 55B can comprise a square or other rectangular shape. As another example, FIG. 8D illustrates an electrode 26 of a flexible polymer circuit strip 24 having decagonal apertures 55C through the covering 54. In this example, fifteen decagonal apertures 55C can be formed through the covering 54 with each decagonal aperture 55C being spaced equally from each other. As yet another example, FIG. 8E illustrates an electrode 26 of a flexible polymer circuit strip 24 having triangular apertures 55D through the covering 54. In this example, nineteen triangular apertures 55D can be formed through the covering 54. The triangular apertures 55D can be offset between each row of triangular apertures 55D such that a first row comprises four triangular apertures 55D while a second row comprises three triangular apertures 55D. Further, alternating rows can be inverted in relation to the one previous. This can allow for partially nesting of the tip of the inverted triangular aperture 55D between two other triangular apertures 55D in the previous row. The coating 27 can be adhered to the exposed surface of the electrode 26 within each rectangular aperture 55B, decagonal aperture 55C, triangular aperture 55D, etc.

As will be appreciated by one of skill in the art, apertures 55 of various other shapes and sizes can be formed through the covering 54 to expose the surface of the electrode 26. Furthermore, apertures 55 of various shapes can be formed through the covering 54 over a single electrode 26. For example, circular apertures 55A, decagonal apertures 55C, and triangular apertures 55D can be formed together over a single electrode 26. Similarly, apertures 55 of one size can be formed through the covering 54 over an electrode 26 along with apertures 55 of a different size. Further still, the apertures 55 may be equally spaced across the surface of the electrode 26 or unequally spaced across the surface of the electrode 26.

FIGs. 8F and 8G illustrate example electrodes 26 of a flexible polymer circuit strip 24 having apertures 55 which are elongated slits 55E, 55F formed through the covering 54. At least four elongated slits 55E, 55F can be formed through the covering 54 to expose the surface of the electrode 26, although it will be appreciated that more or fewer elongated slits 55E, 55F can be formed depending on the application. In the example illustrated in FIG. 8F, the elongated slits 55E can extend from near one end of the electrode 26 to near a second end of the electrode 26 in a lengthwise direction. In the example illustrated in FIG. 8G, the elongated slits 55E can extend from near one end of the electrode 26 to near a second end of the electrode 26 in a widthwise direction.

As will be appreciated by one of skill in the art, by forming elongated slits 55E, 55F through the covering 54, a greater continuous surface area of the electrode 26 may be exposed which can help to increase the exposed conductive surface area of the electrode 26 but may also increase the likelihood of the coating 27 being rubbed while in use. Therefore, the spacing and size of the elongated slits 55E, 55F can be varied to help ensure the electrode 26 has a sufficient amount of surface area exposed while also ensuring the coating 27 is sufficiently protected.

FIG. 8H illustrates an example electrode 26 of a flexible polymer circuit strip 24 having apertures 55 which are elongated slits 55G formed through the covering 54. Unlike the elongated slits 55E, 55F illustrated in FIGs. 8F and 8G, the elongated slits 55G extend only a portion of the length of the electrode 26 (e.g., approximately less than 1/3 of the length of the electrode 26). In this way, the elongated slits 55G can be configured to provide greater mechanical protection to the coating 27 but still ensure a sufficient amount of the electrode 26 is exposed.

FIG. 8I illustrates an example electrode 26 of a flexible polymer circuit strip 24 having a combination of circular apertures 55A and elongated slits 55E. In this example, the elongated slits 55E can help to increase the exposed surface area of the electrode 26 while the circular apertures 55A can expose some of the surface area of the electrode 26 while also helping to provide greater mechanical protection to the coating 27. As will be appreciated by one of skill in the art, any of the example apertures 55A-55D and elongated slits 55E-55G can be combined to help ensure a sufficient amount of the electrode 26 is exposed while also ensuring the coating 27 is suitably protected.

Reference is now made to FIG. 8J, which is a table (Table 1) illustrating impedance values for example patterns of apertures 55 formed in the covering of the flexible polymer circuit strips of FIG. 7. Although Table 1 illustrates impedance values that were experimentally obtained for a few selected patterns of apertures 55, impedance values may also be obtained for any of the patterns of apertures 55 described herein. Thus, Table 1 should not be construed as limiting but is offered to illustrate the impedance values of a few example patterns of apertures 55.

As illustrated in FIG. 8J, the impedance values (in ohms) for six different aperture 55 patterns and two control samples (one with coating 27 covering approximately 100% of the electrode 26 surface and one without any coating 27) are shown at frequencies of 1 Hz, 10 Hz, 50 Hz, and 100 Hz. As illustrated, as the input frequency increases, the impedance generally decreases. Furthermore, the impedance values are inversely related to the exposed surface area. Illustrations of the six different aperture 55 patterns are shown below Table 1 for explanatory purposes.

Starting from left to right in table 1, impedance data of a first example electrode 26 (Example 1) having three rows of seven circular apertures 55A in each row is shown. The impedance of Example 1 can range from approximately 10,406 ± 920 ohms at 1 Hz to approximately 168 ± 28 ohms at 100 Hz. Example 2 similarly illustrates an electrode 26 having circular apertures 55A, however, Example 2 comprises two rows of five circular apertures 55A in each row. As shown, the impedance of Example 2 can range from approximately 12,502 ± 552 ohms at 1 Hz to approximately 206 ± 20 ohms at 100 Hz. As will be appreciated, because Example 2 has less surface area of the electrode 26 coated with the coating 27, the covering 54 covers a greater amount of the surface area of the electrode 26 and can be more mechanically robust since more covering 54 material can be located between each circular aperture 55A.

Continuing from left to right in Table 1, Example 3 illustrates an electrode 26 having four elongated slits 55E stretching from near one end of the electrode 26 to near a second end of the electrode 26. The impedance of Example 3 can range from approximately 7,000 ± 467 ohm at 1 Hz to approximately 109 ± 4 ohms at 100 Hz. Example 4 illustrates an electrode 26 having three rows of elongated slits 55G with each elongated slit 55G extending only a portion of the electrode 26 surface. In particular, Example 4 comprises three rows of three elongated slits 55G. The impedance of Example 4 can range from approximately 10,544 ± 235 ohms at 1 Hz to approximately 164 ± 8 ohms at 100 Hz. As will be appreciated, because the elongated slits 55G of Example 4 extend only a portion of the surface of the electrode 26, the coating 27 can be more mechanically protected by the covering 54 when compared to Example 3.

Example 5 and Example 6 in Table 1 illustrate electrodes 26 having an aperture 55 sized to expose approximately one-third and two-thirds of the electrode 26 respectively. As shown, the impedance value of Example 5 can range from approximately 16,921± 4,158 ohms at 1 Hz to 306 ± 77 ohms at 100 Hz while the impedance value of Example 6 can range from approximately 9,951 ± 407 ohms at 1 Hz to 186 ± 24 ohms at 100 Hz. As will be appreciated, although the impedance may be reduced by having a larger aperture size 55 as shown in Example 6, the coating 27 may have a greater tendency of being damaged because the covering 54 is less able to provide mechanical protection to the coating 27.

In the two far right columns of Table 1, impedance values for two control examples are included for reference. First, a control showing an electrode 26 having approximately 100% of its surface coated with the coating 27 is shown. In this example, the overall impedance can range from approximately 6,629 ± 197 ohms at 1 Hz to 117 ± 3 ohms at 100 Hz. In the second control example, an electrode having none of its surface coated with the coating 27 is shown. The impedance values for an electrode 26 not having any coating 27 can range from approximately 265,513 ± 9,186 ohms at 1 Hz to 3,636 ± 182 ohms at 100 Hz. As these two control examples illustrate, the coating 27 can help to significantly reduce the overall impedance of the electrode 26. However, as previously explained, the coating 27 can become damaged and eventually delaminate if the coating 27 is impacted by components of the basket catheter 10 or other objects. Thus, by forming apertures 55 through the covering 54 and then coating the electrode's 26 surface with the coating 27, the disclosed technology can reduce the overall impedance while also helping to reduce the likelihood of damaging the coating 27.

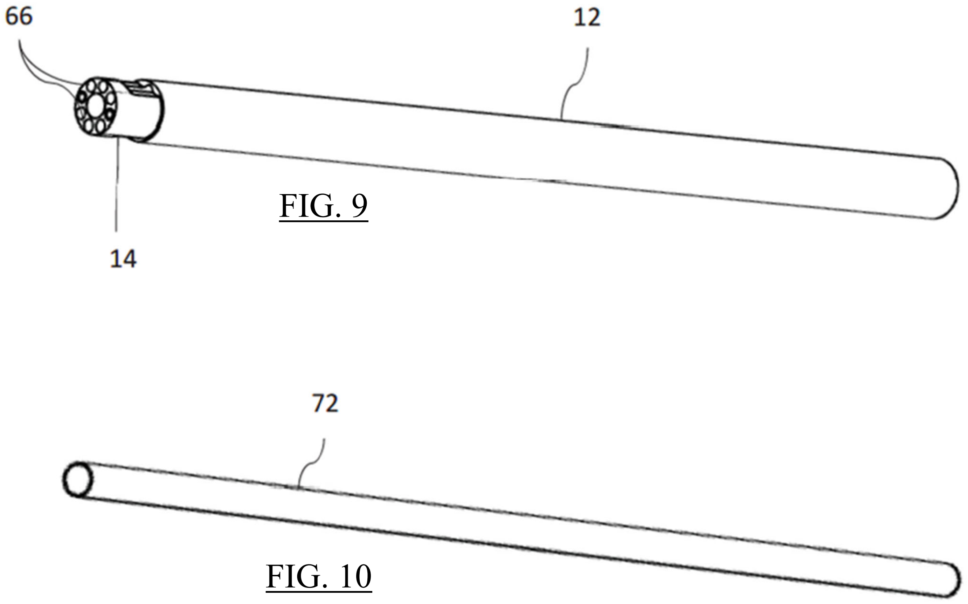

Reference is now made to FIG. 9, which is a schematic view of the elongated deflectable element 12 of the basket catheter 10 of FIGs. 1A and 1B. The elongated deflectable element 12 may be produced from any suitable material, for example, polyurethane or polyether block amide. The distal end 14 of the elongated deflectable element 12 has a smaller outer diameter than the rest of the elongated deflectable element 12 to accept the coupler 16 thereon as shown in FIG. 20. The elongated deflectable element 12 includes lumens 66 for inserting various tubes and wires therein as described herein. The elongated deflectable element 12 may have any suitable outer diameter and length, for example, the outer diameter may be in a range between 1 mm and 4 mm and the length may be in a range between 1 cm and 15 cm.

Reference is now made to FIG. 10, which is a schematic view of an irrigation sleeve 72 of the basket catheter 10 of FIGs. 1A and 1B. The irrigation sleeve 72 is a flexible tube which is disposed in one of the lumens 66 (FIG. 9) of the elongated deflectable element 12 (FIG. 9). The irrigation sleeve 72 may be used to carry irrigation fluid to the region of the expandable assembly 22 (FIGs. 1A and 1B). The irrigation sleeve 72 is sized to fit in one of the lumens 66 (typically a central lumen) of the elongated deflectable element 12 and extend beyond the distal end 14 (FIG. 9) of the elongated deflectable element 12 as shown in FIG. 20. The inner and outer diameter of the irrigation sleeve 72 may be in the range between 3mm and 5mm. The irrigation sleeve 72 may be formed from any suitable material, for example, but not limited to polyimide, polyurethane, polyether block amide, or polyethylene terephthalate.

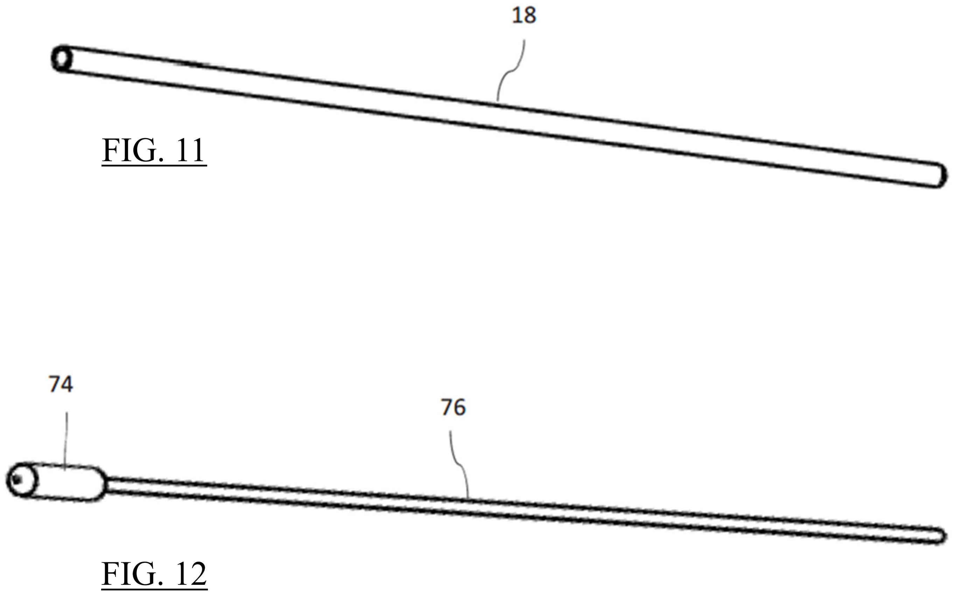

Reference is now made to FIG. 11, which is a schematic view of the pusher 18 of the basket catheter 10 of FIGs. 1A and 1B. The pusher 18 is a flexible tube and is disposed in the irrigation sleeve 72. The pusher 18 is sized to slide in the irrigation sleeve 72 and allow room for irrigation fluid to pass between the irrigation sleeve 72 and the pusher 18. The inner diameter of the pusher 18 is sized to accommodate wiring of a multi-axis position sensor described with reference to FIG. 12. The pusher 18 extends beyond the distal end 14 of the elongated deflectable element 12 (FIG. 9) until the nose connector 30 as shown in FIG. 19. The pusher 18 may be formed from any suitable material, for example, but not limited to polyimide with or without braiding, polyether ether ketone (PEEK) with or without braiding, or polyamide with or without braiding.

Reference is now made to FIG. 12, which is a schematic view of a multi-axis position sensor 74 of the basket catheter 10 of FIGs. 1A and 1B. The multi-axis position sensor 74 may comprise a dual-axis or triple-axis position sensor, for example, a magnetic position sensor comprising multiple orthogonal coils. Wiring 76 is used to connect the multi-axis position sensor 74 via the hollow of the pusher 18 (FIG. 11) to a position computation system (not shown) disposed proximally to the basket catheter 10. The multi-axis position sensor 74 and the wiring 76 are shown in more detail in FIGs. 5 and 19.

Reference is now made to FIGs. 13A-B, which are schematic views of the nose connector 30 of the basket catheter 10 of FIGs. 1A and 1B. The nose connector 30 may be formed from any suitable material, for example, but not limited to polycarbonate with or without glass filler, PEEK with or without glass filler, or PEI with or without glass filler. The nose connector 30 includes a proximal cavity 78 (FIG. 13A) in which the pusher 18 (FIG. 11) is secured and through which the wiring 76 passes as shown in FIG. 19. FIG. 13B also shows the distal receptacle 34, the inner surface 36, and the distal facing opening 38. The distal receptacle 34 houses the multi-axis position sensor 74 (FIG. 12) and the hinges 28 (FIG. 5) which are connected to the inner surface 36.

Reference is now made to FIG. 14, which is a schematic view of the center electrode ring 40 of the basket catheter 10 of FIGs. 1A and 1B. Electrode 40 is electrically connected to a wire (not shown) that passes through the slot in the side of proximal cavity 78 and into pusher 18. The center electrode ring 40 may be formed from any suitable material, for example, but not limited to noble metals and their alloys comprising platinum, palladium, gold, or iridium. The center electrode ring 40 serves a secondary role by providing mechanical support around the proximal cavity 78 (FIG. 13A) of the nose connector 30 to secure the nose connector 30 to the pusher 18 (FIG. 11) as shown in FIG. 19.

Reference is now made to FIGs. 15A-B, which are schematic views of the nose cap 32 of the basket catheter 10 of Fig 1. The nose cap 32 includes a hollow cylinder 80 covered with a cover 82 which may be wider than the hollow cylinder 80. The nose cap 32 may be formed from any suitable material, for example, but not limited to polycarbonate with or without glass filler, PEEK with or without glass filler, or PEI with or without glass filler. The nose cap 32 is sized to fit in the distal receptacle 34 (FIG. 13B) of the nose connector 30 (FIG. 13B) and cover the distal facing opening 38 (FIG. 13B) while allowing space for the multi-axis position sensor 74 (FIG. 12) and the hinges 28 (FIG. 5) therein as shown in FIG. 19. The nose cap 32 may optionally be sized to provide a pressure fit against the hinges 28 to prevent the hinges 28 from being pulled away from the inner surface 36 (FIG. 13B) of the nose connector 30 (FIG. 13B). The nose connector 30 may also function to protect the multi-axis position sensor 74.

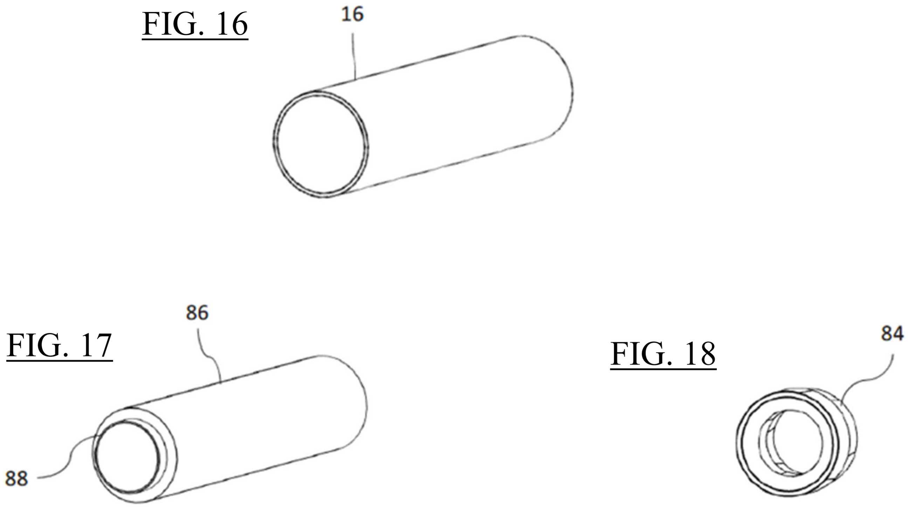

Reference is now made to FIG. 16, which is a schematic view of the coupler 16 of the basket catheter 10 of FIGs. 1A and 1B. The coupler 16 typically comprises a hollow tube and may be formed from any suitable material, for example, but not limited to polycarbonate with or without glass filler, PEEK with or without glass filler, polyimide, polyamide, or PEI with or without glass filler. The coupler 16 may be sized to have the same inner diameter as the outer diameter of the distal end 14 (FIG. 9) of the elongated deflectable element 12 (FIG. 9) and the same outer diameter as the proximal portion of the elongated deflectable element 12. The coupler 16 is also sized to surround various elements described in more detail with reference to FIG. 20.

Reference is now made to FIG. 17, which is a schematic view of a single-axis position sensor 86 of the basket catheter 10 of FIGs. 1A and 1B. The single-axis position sensor 86 may include any suitable position sensor, for example, a magnetic position sensor comprising a coil wound on a hollow cylinder 88. Wiring (not shown) from the single-axis position sensor 86 may be passed down one of the lumens 66 (FIG. 9) to a position computation system (not shown) disposed proximally to the basket catheter 10. The hollow cylinder 88 is sized to accommodate the irrigation sleeve 72 therein as shown in FIG. 20. The outer diameter and length of the single-axis position sensor 86 is sized to fit in the coupler 16 (FIG. 16). The hollow cylinder 88 may be formed from any suitable material, for example, but not limited to, a material used as a magnetic core.

Reference is now made to FIG. 18, which is a schematic view of a proximal retainer ring 84 of the basket container 10 of FIGs. 1A and 1B. The proximal retainer ring 84 is configured to provide a pressure fit around the distal end of the irrigation sleeve 72 (FIG. 10) and retain the single-axis position sensor 86 (FIG. 17) to be adjacent to the distal end 14 (FIG. 9) of the elongated deflectable element 12 (FIG. 9) as shown in FIG. 20. The proximal retainer ring 84 also serves to secure the flexible polymer circuits 24 between the retainer ring 84 and the coupler 16. The proximal retainer ring 84 may be formed from any suitable material, for example, but not limited to polycarbonate with or without glass filler, PEEK with or without glass filler, or PEI with or without glass filler.

Reference is now made to FIGs. 19-20, which are cross sectional views through line A-A of FIG. 1B. FIG. 19 shows a distal portion of the expandable assembly 22, while FIG. 20 shows a proximal portion.

FIG. 19 shows that the distal portion 20 of the pusher 18 is disposed in the proximal cavity 78 of the nose connector 30 and is secured therein using the center electrode ring 40 disposed around the outside of the proximal cavity 78. The multi-axis position sensor 74 is disposed in the distal receptacle 34 of the nose connector 30 with the wiring 76 extending proximally through the pusher 18. The second ends 46 of the flexible polymer circuit strips 24 are connected to the inner surface 36 of the distal receptacle 34 of the nose connector 30. The elongated resilient support elements 48 extend along the length of the flexible polymer circuit strips 24 until, but not including, the hinges 28. The nose cap 32 is inserted into the distal receptacle 34 with the hollow cylinder 80 surrounding the distal portion of the multi-axis position sensor 74 and providing pressure against the second ends 46 of the flexible polymer circuit strips 24. The nose cap 32 covers the distal facing opening 38 of the nose connector 30.

FIG. 20 shows that the irrigation sleeve 72 is disposed in the elongated deflectable element 12. The pusher 18 is disposed in the irrigation sleeve 72. The wiring 76 is disposed in the pusher 18. The single-axis position sensor 86 is disposed around the irrigation sleeve 72 (between the coupler 16 and the pusher 18) close to the distal end 14 of the elongated deflectable element 12. The proximal retainer ring 84 provides a pressure fit around the irrigation sleeve 72 and keeps the single-axis position sensor 86 in place distally to the distal end 14 of the elongated deflectable element 12. The proximal end of the coupler 16 is connected to the distal end 14 of the elongated deflectable element 12. The first ends 42 of the flexible polymer circuit strips 24 are connected to the inner surface 44 of the coupler 16. FIG. 20 shows that the elongated resilient support elements 48 extend along the respective strips 24 from the coupler 16 until before the respective hinges 28 (FIG. 19).

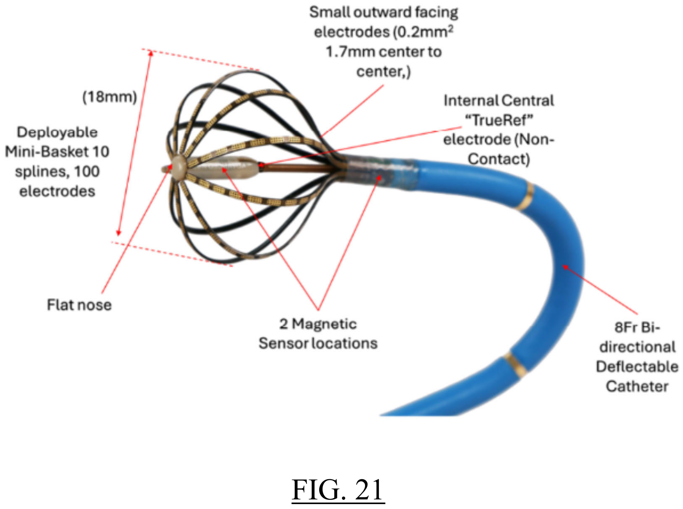

FIG. 21 illustrates another view of the catheter 10 with annotations illustrating various features of the catheter 10. For example, FIG. 21 illustrates the expandable assembly 22 can be sized to expand to a diameter of approximately 18 mm, can include 10 spines 24 with 10 electrodes 26 each (100 electrodes 26 total), have a small outward facing electrodes being sized to approximately 0.2 mm² spaced at 1.7 mm center to center, include an internal central "TruRef" electrode (reference electrode 31), the approximate locations of a first and a second position sensor 74, and that the elongated deflectable element 12 can be approximately 8 French in size and be bi-directional deflectable.

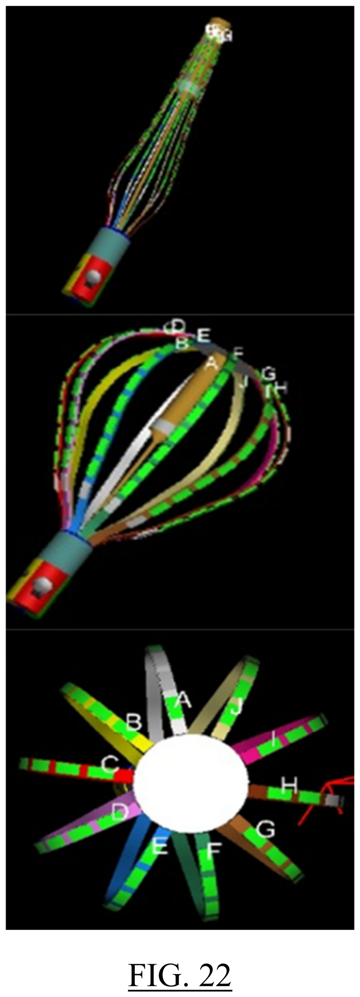

FIG. 22 illustrates views of the basket catheter 10 as rendered on a display showing the basket in an expanded and in a collapsed state. As shown, the basket catheter 10 can be graphically displayed with various colors and annotations to indicate various orientations and specific spines to help indicate to the physician 5 the orientation of the catheter 10 within a patient's heart.

While the expandable assembly 22 is shown without being mounted to a flexible membrane, it is within the scope of the invention that the expandable assembly can be provided with a membrane (e.g., balloon like surface) as a base substrate for the circuit strips. As well, the membrane can be used as a covering layer over the circuit strips 24 with electrodes 26 being exposed (or not covered by the membrane for exposure) to the ambient environment (e.g., inside organ tissues).

### Description of Methods and Study

This disclosure is more clearly understood with the corresponding studies and methods described herein. It is understood that data is presented herein for purposes of illustration and should not be construed as limiting the scope of the disclosed technology in any way or excluding any alternative or additional embodiments.

The catheter 10 describe above was tested by physicians in a prospective, single-arm, multicenter study included patients undergoing catheter mapping and ablation of atrial and ventricular arrhythmias. Mapping was performed with the study catheter 10 and participants were ablated per investigator's standard-of-care. The primary effectiveness endpoint was completion of pre-ablation electro-anatomical mapping without resorting to a non-study catheter. The primary safety endpoint was incidence of device-related serious adverse events (SAE) within 7 days. Physician feedback on catheter performance was collected via a 7-point Likert scale.

As part of the study, forty participants (mean age 58.0±15.73 years, 62.5% male, 30 with atrial arrhythmias, 10 with ventricular arrhythmias) underwent mapping with the study catheter. The primary effectiveness endpoint was achieved in all 40 participants; ≥1 area of interest for mappable rhythms was identified in 23/30 atrial arrythmia participants. Only 1 SAE of transient complete atrioventricular block was reported in a persistent atrial fibrillation patient during pre-ablation mapping, with full recovery. Physician feedback indicated the device met or exceeded expectation for signal quality; most responders rated highly on bipolar signal quality in atria and noise encountered.

In this first-in-human clinical study, the investigational ultra-high-density globe-shaped catheter achieved its primary safety and efficacy endpoints with a favorable acute safety and effectiveness profiles for mapping complex arrhythmias. With its deflectable shaft and variable basket deployment, the catheter was able to access all chambers of the heart. For all study procedures, protocol-required pre-ablation mapping was completed with the investigational catheter with no requirement to switch to another mapping catheter. The safety profile of the investigational catheter was comparable to that experienced with other commercially available high-density mapping catheters used for atrial and ventricular procedures. Only one SAE within seven days of index procedure was deemed related to the investigational catheter; three procedure-related SAEs were unrelated to the catheter, while one non-serious AE was considered related to the investigational catheter. For all events, participants fully recovered and were discharged within seven days after the procedure. Mean pre-ablation mapping time was 23.8, 21.6, 26.8, 5.4, and 17.9 minutes for participants with AT/AFL, PsAF, PAF, VT, and PVC respectively.

Like other high-density mapping catheters, the investigational catheter detected PVI breakthroughs and triggers. The investigational catheter's design facilitated identifying mechanisms sustaining arrhythmias quickly, enhancing procedural workflow and efficiency. The presence of three embedded magnetic sensors in the distal and proximal portion of the globe that transmit information to the CARTO^{®} 3 System allowed for basket visualization in an open or closed shape. In addition, the location and angle information of the catheter can be transferred to the CARTO^{®} 3 System independently of advanced catheter location. Moreover, the combination of an increased number of close-spaced small flat electrodes and internal TRUEref reference electrode offered greater mapping density with a high intracardiac signal resolution. Experience in the current study built on preclinical observations that TRUEref electrode was able to filter out far field ventricular signals during atrial mapping and around lines of block and reduce incorrect timing annotations. The wisdom of crowd algorithm was able to reduce outlier misannotated LAT points based on a certain criterion collected within a 1-mm³ pre-defined FAM area (voxel).

Overall, operators rated the investigational catheter as equal to or an improvement over the PENTARAY^{®} catheter in confirming PVI, its arrhythmogenicity, and tissue characterization. The study was limited, however, by having a small sample size of participants spread across different arrhythmia subgroups. Participants with the most common arrhythmia mechanisms were expected to enroll in the study rather than a range of arrhythmias. Ideally, a greater proportion of ischemic VT cases could have added to a fuller assessment of the investigational catheter, and larger studies with a longer follow-up would be a better indication of effectiveness. CARTO^{®} V7 was the latest CARTO^{®} version available at the time of this study. Other unique features designed to leverage the investigation catheter's globe-shape design were not yet available during the period of the study procedures.

### Study Endpoints

The primary effectiveness endpoint was the completion of protocol-required electro anatomical pre-ablation mapping without resorting to non-study mapping catheters. The primary safety endpoint was the incidence of device-related serious adverse events (SAE) within seven days following the procedure.

The secondary effectiveness endpoint was the deployment characterization, maneuverability, and signal quality acquired with the investigation catheter in the atria and ventricles based on physicians' feedback on the post-procedure survey. The survey was collected after each study procedure for each investigation catheter used and included questions with Likert-scale response options for maneuverability and handling, signal collection and quality, pacing, catheter design, workflow, visualization, catheter's interactions, arrhythmogenicity, design and coverage for confirming PVI, and ability to characterize the tissue. A score of "4" on the 7-point scale was considered comparable to other devices. The secondary safety endpoint was the incidence of SAEs excluding investigational catheter-related SAE within seven days of index procedure and incidence of non-serious adverse events (AE) within seven days of index procedure related to the investigational catheter.

Additional procedural characteristics were total procedure time, initial mapping duration (time between first and last mapping point prior to first ablation point, as measured on CARTO), areas of interest captured (e.g. PV triggers, previous PVI lesion gaps, slow conduction scar zone, critical isthmus, etc.), and mapping density.

Arrythmia mapping was assessed as an additional endpoint. An atrial reference was used to determine the cycle length of atrial tachycardias (ATs). The window was adjusted to include points across the entire atrial cycle length or pre-P-wave for atrial flutter (AFL) or focal AT, respectively, and automatic points acquisition (CONFIDENSE) was set to the individual operator's discretion. A ventricular reference was used to determine the cycle for ventricular tachycardias (VTs) or mapping of premature ventricular complexes (PVCs). The window was adjusted to include points across the entire ventricular cycle length or pre QRS wave for sustained VTs or PVCs, respectively, and automatic points acquisition (CONFIDENSE) set to the operator's discretion.

### Study Description

Eligible participants were scheduled to have a clinically indicated catheter mapping and ablation procedure for VT, PVC, AT, AFL, or paroxysmal or persistent atrial fibrillation (PAF or PsAF); patients having undergone a previous ablation procedure could be included. Exclusion criteria included diagnosis of an arrhythmia requiring epicardial mapping, left ventricular ejection fraction (LVEF) ≤25% for patients with VT, and LVEF ≤40% for patients with atrial arrhythmia.

Pre-procedure assessment and data collection included baseline medical, cardiac, arrhythmic and ablation history, transthoracic echocardiogram, pregnancy test, thrombus screening, collection of any AEs since enrollment, and subsequent ablation according to institutional standard of care (SOC) practice.

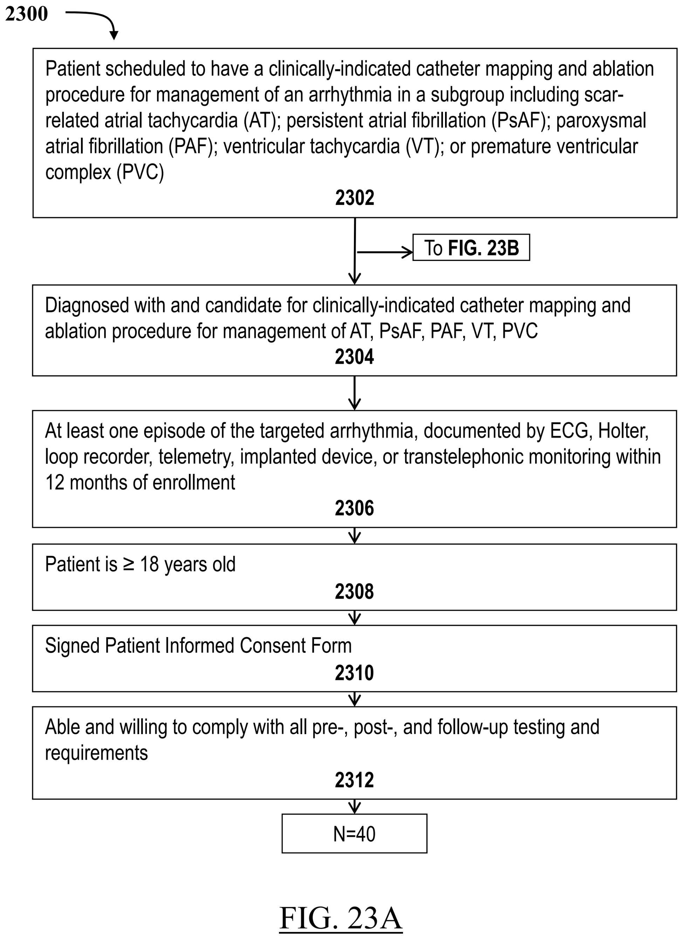

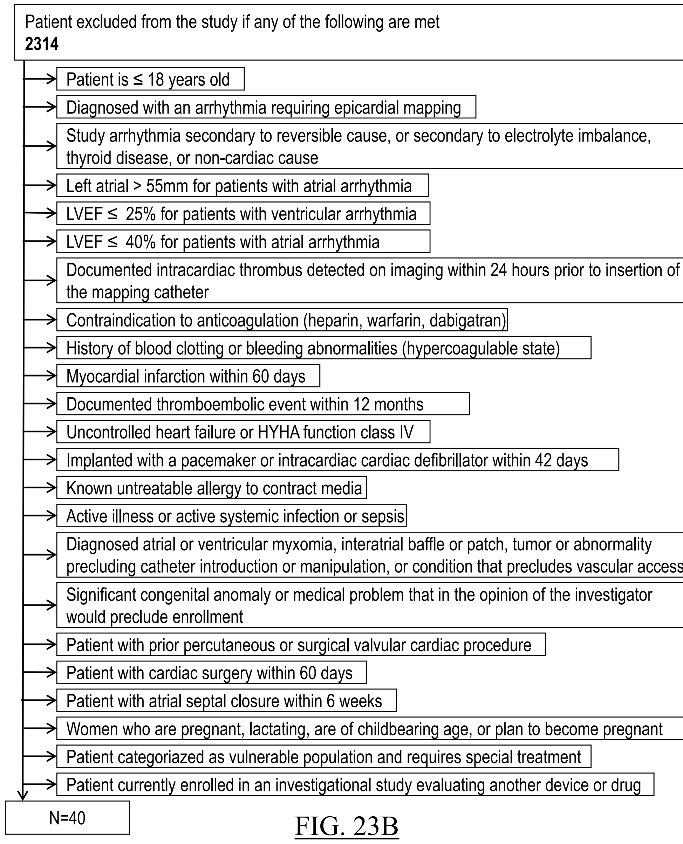

FIGs. 23A and 23B illustrate further details of the inclusion and exclusion criteria for patients as part of a study of the catheter, in accordance with an example of the present invention. As shown, participants in the study must qualify for the study by meeting various requirements such as being scheduled 2302 to have a clinically-indicated catheter mapping and ablation procedure for management of an arrhythmia in a subgroup including scar-related atrial tachycardia (AT); persistent atrial fibrillation (PsAF); paroxysmal atrial fibrillation (PAF); ventricular tachycardia (VT); or premature ventricular complex (PVC). If the patients meets this first requirement, the patient must also be diagnosed 2304 with and candidate for clinically-indicated catheter mapping and ablation procedure for management of AT, PsAF, PAF, VT, PVC.

To be included in the study, the patient must also have at least one episode 2306 of the targeted arrhythmia, documented by ECG, Holter, loop recorder, telemetry, implanted device, or transtelephonic monitoring within 12 months of enrollment, be greater than or equal to 18 years old 2308, sign a patient informed consent form 2310, and be able 2312 and willing to comply with all pre-, post-, and follow-up testing and requirements. This study included 40 participants.

Participants were excluded 2314 from the study if any of the following criteria applied: patient was less than 18 years old, diagnosed with an arrhythmia requiring epicardial mapping, study arrhythmia secondary to reversible cause, or secondary to electrolyte imbalance, thyroid disease, or non-cardiac cause, atrial arrhythmias: patients with a left atrial size >55 mm, LVEF ≤ 25% for patients with ventricular arrhythmia, LVEF ≤ 40% for patients with atrial arrhythmia, patient had documented intracardiac thrombus as detected on imaging within 24 hours prior to insertion of the investigational catheter, contraindication to anticoagulation (i.e. heparin, warfarin, dabigatran), history of blood clotting or bleeding abnormalities (e.g. hypercoagulable state), myocardial infarction within the past 2 months (60 days), documented thromboembolic event (including TIA) within the past 12 months (365 days), uncontrolled heart failure or NYHA function class IV, implanted with a pacemaker or intracardiac cardiac defibrillator within the past 6 weeks (42 days), patients with known untreatable allergy to contrast media, active illness or active systemic infection or sepsis, diagnosed atrial or ventricular myxoma, interatrial baffle or patch, tumor or other abnormality that precludes catheter introduction or manipulation, significant congenital anomaly or medical problem that in the opinion of the investigator would preclude enrollment in this study, subjects that have ever undergone a percutaneous or surgical valvular cardiac procedure (i.e., ventriculotomy, atriotomy, and valve repair or replacement and presence of a prosthetic valve), any cardiac surgery within the past 60 days (2 months) (includes PCI), atrial septal closure within the past 6 weeks (42 days), presence of a condition that precludes vascular access, women who are pregnant (as evidenced by pregnancy test if pre-menopausal), lactating, or who are of childbearing age and plan on becoming pregnant during the course of the clinical investigation, patients who are categorized as vulnerable population and requires special treatment with respect to safeguards of well-being, or concurrent enrollment in an investigational study evaluating another device or drug. If a patient met any of the foregoing criteria, the patient was not included in the study.

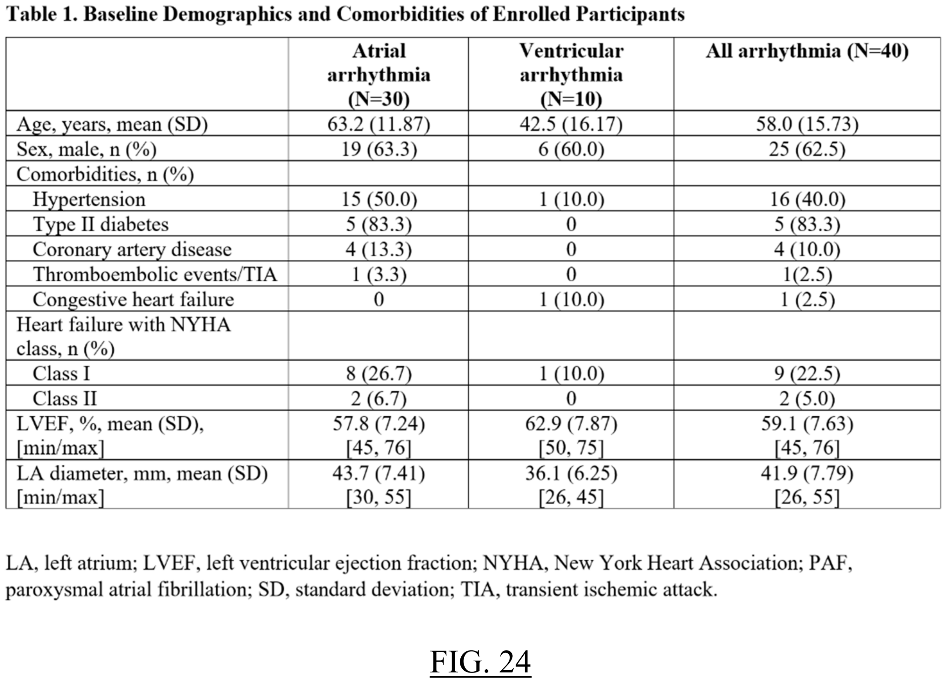

FIG. 24 is a table illustrating the baseline demographics and comorbidities of participants enrolled in the study, in accordance with an example of the present invention. A total 40 participants completed the study procedures. The mean age of participants was 58.0 years and 25 (62.5%) were male. Most participants (38/40, 95.0%) were without structural heart disease. Around two-thirds had no heart failure (27/40, 67.5%), whereas 9 participants (22.5%) had NYHA class I and 2 participants (5.0%) had NYHA class II heart failure. Of the 30 participants with atrial arrhythmia (7 with AFL, 2 with AT, 7 with PsAF, and 14 with PAF), 16 participants (53.3%) had a history of ≥1 prior ablation procedure, which were performed for the treatment of AF (15 participants), typical AFL (5 participants), AT (2 participants), or atypical AFL (2 participants). These prior ablation procedures were performed using radiofrequency (14 procedures), cryoablation (1 procedure), and other ablation technologies (2 procedures). Of the 10 participants with ventricular arrhythmia (1 with VT and 9 with PVC), 2 participants had prior ablations for the treatment of AF (1 patient) and PVC (1 patient), both with radiofrequency catheters.

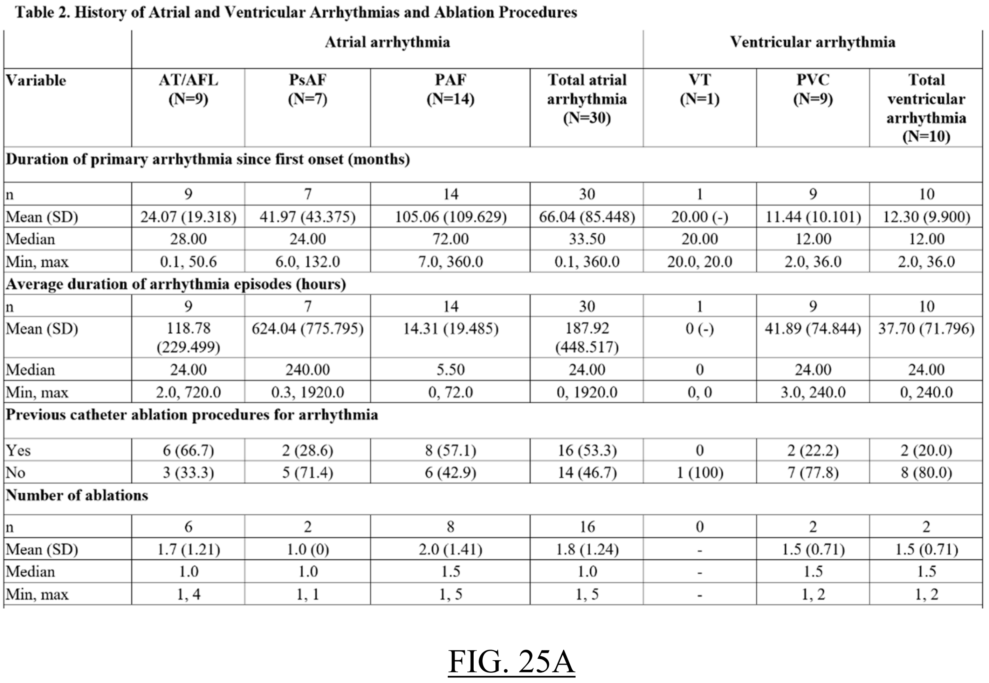

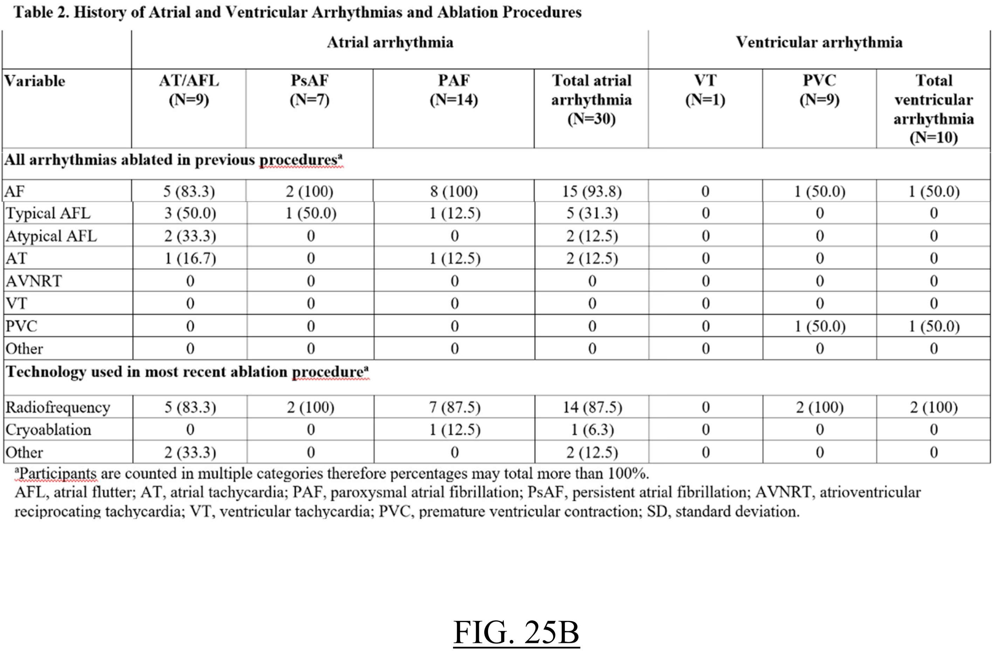

FIGs. 25A and 25 B is a table of history of atrial and ventricular arrhythmias and ablation procedures of participants enrolled in the study, in accordance with an example of the present invention.

All participants (40/40, 100%) completed the protocol-required pre-ablation mapping with the investigational catheter. The entire chambers of interest targeted for arrhythmia mapping were completed with FAM. No other non-study mapping catheter was used for any pre-ablation mapping.

The investigational catheter was used solely for all pre-ablation mapping procedures. The catheter was advanced into the cardiac chamber of interest via any commercially available 8.5Fr sheath and maps were created with automatic acquisition (CONFIDENSE) of points for each beat gated to respiratory and cardiac cycle. Pre-ablation mapping included FAM of the entire chamber(s) and areas associated with targeted arrhythmia. Electro-anatomical mapping was performed to determine substrate voltage or tachycardia activation mechanism, local activation timing (LAT), identification of conduction channel, gap(s) and critical isthmus, and determination of adequate level of mapping density at the area of interest. The investigational catheter was used with continuous irrigation and activated clotting time of ≥300 seconds. Heparinized saline (1 unit/mL) was infused through the central lumen of the catheter shaft at a rate of 2 mL/min, emerging at the end of the shaft (proximal end of the basket) to prevent thrombus.

After mapping, the ablation procedure was performed as per the institution's standard of care. If additional mapping was clinically indicated after ablation, the investigational catheter was used. Phrenic nerve pacing was frequently performed during catheter ablation to assess any nerve damage by the ablation catheter. The ability of the study catheter in conducting phrenic nerve pacing or pacing capture was examined. The follow-up period was 7 days with a telephone call or an in-person clinic visit to assess for AEs.

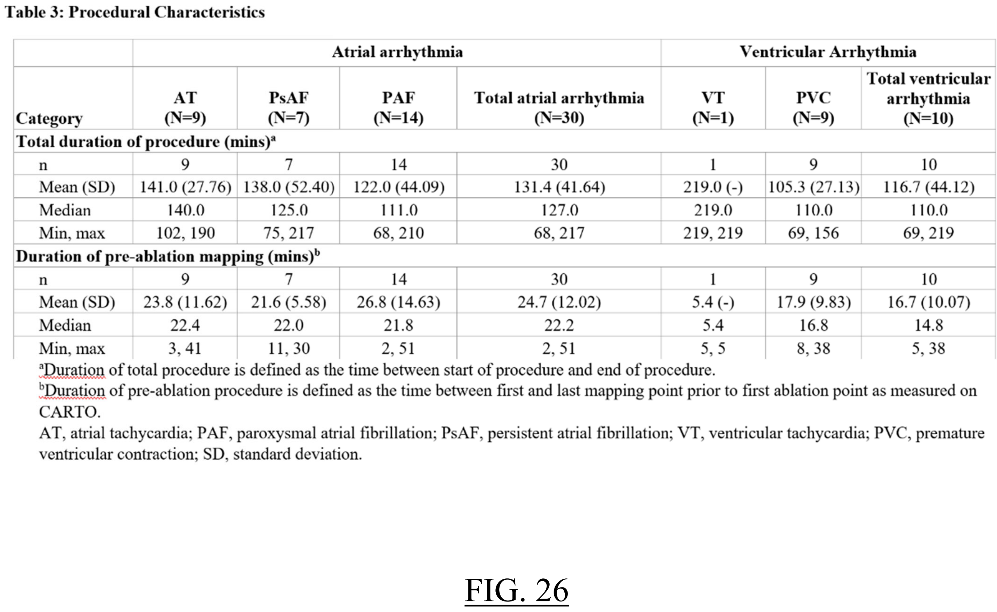

FIG. 26 is a table of procedural characteristics of the procedures completed as part of the study, in accordance with an example of the present invention. For atrial procedures, mean total procedural duration was 131.4 minutes (141.1 minutes for participants with AT/AFL, 138.0 minutes for participants with PsAF, and 122.0 minutes for participants with PAF), while mean total pre-ablation mapping time was 24.7 minutes (23.8 minutes for participants with AT/AFL, 21.6 minutes for participants with PsAF, and 26.8 minutes for participants with PAF). For ventricular procedures, mean total procedural duration was 116.7 minutes (219.0 minutes for the 1 patient with VT and 105.3 minutes for the PVC participants), while mean total pre-ablation mapping time was 16.7 minutes (5.4 minutes for the patient with VT and 17.9 minutes for participants with PVC) (FIG. 26).

FAM maps were created in all participants, with voltage maps created in all 30 atrial procedures and 8/10 ventricular procedures. In addition, LAT maps were created in 25/30 atrial procedures and 7/10 ventricular procedures. The investigational catheter was used for phrenic nerve pacing in 5 participants, with local pacing capture demonstrated in all of these procedures. Phrenic nerve stimulation was not performed in any procedure. In addition, 30/40 participants (75.0%) had post-SOC mapping performed.

No embolic events were reported. At the end of the procedure, catheters were inspected, and no visible thrombus, device malfunctions, or cardiac structure entanglement were reported.

In all 30 atrial arrythmia procedures, FAM and voltage maps were completed. LAT mapping was performed in 83.3% (25/30) of participants, including all 9 focal AT/AFL procedures (100%), 11/14 PAF procedures (78.6%), and 5/7 PsAF procedures (71.4%). FAM maps were also created in 100% (10/10) of ventricular tachycardia participants. Voltage mapping was performed in 80.0% (8/10) of the subjects and LAT in 70.0% (7/10) of participants.

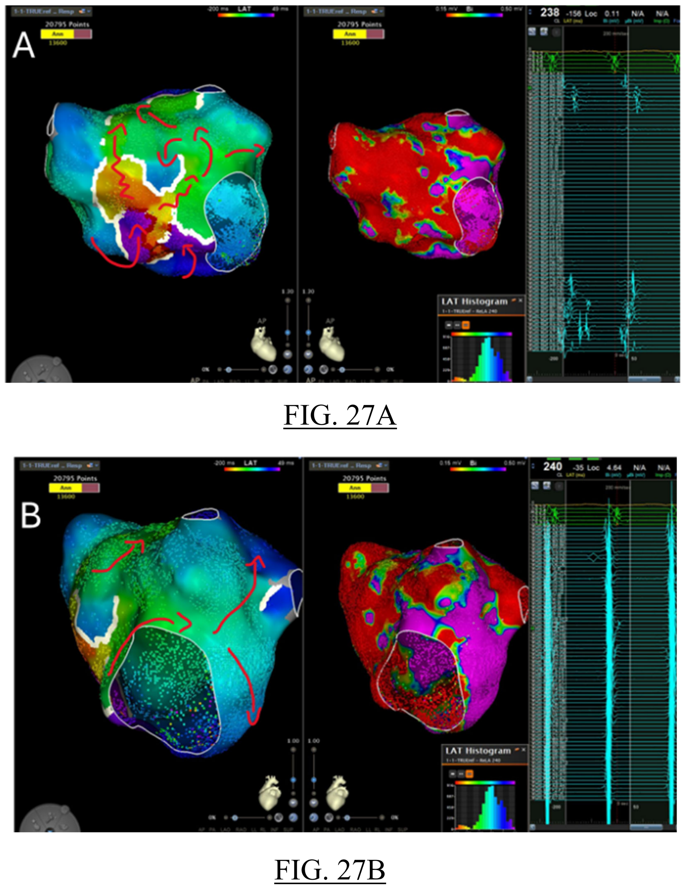

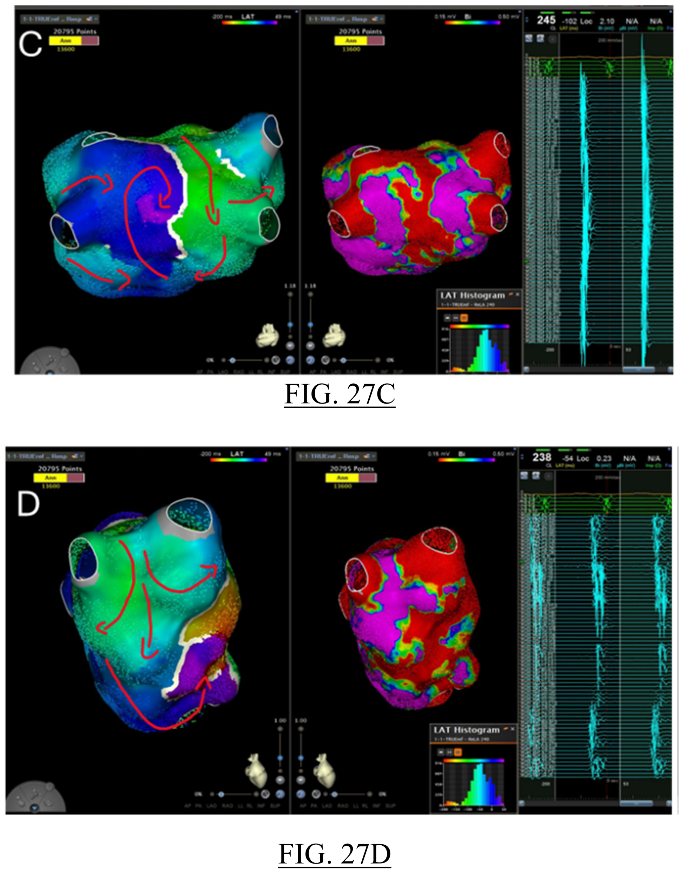

FIGs. 27A-27D represent a graphical representation of a left atrial atypical flutter timing (LAT) and voltage (bipolar) maps of a patient's heart using the catheter, in accordance with an example of the present invention. Some of the images in FIGs. 27A-27D the pathway of the wave propagation has been annotated on the image. The entire tachycardia cycle length (~240ms) was mapped and the circuit identified. Areas of low voltage or scar were also defined with adjusted voltage cutoff (<0.1 mV in red and >0.5 mV in purple). As shown in FIGs. 27A-27 D, the location and timing of electrophysiological signals propagating through the tissue can be mapped and displayed on a display for the physician to see to help identify a location for ablation.

The high-density LAT mapping helped to identify and visualize atypical tachycardia mechanisms. The TRUEref^{™} Electrode (reference electrode 31) on the catheter allowed operators to acquire points with a clearer signal quality, therefore, signals were annotated to the correct nearfield component instead of far field. Also, the wisdom of the crowd algorithm effectively and automatically reduced outlier LAT annotation based on the density of data collected within a 1-mm3 voxel (see FIGs. 27A-27D).

The disclosed technology can include the ability to filter the electrophysiological signals to better identify and display locations of interest. For example, the coloring and tolerances can be adjusted (e.g., via a toggle switch or manual changes to settings in the software) to better highlight a focus of an arrythmia and surrounding area that a physician would want to ablate. As will be described in greater detail herein, the disclosed technology can include a method of identifying and highlighting locations where the activation time is within a predetermined time duration from an earliest activation (e.g., less than one second, less than 10 milliseconds, less than 1 millisecond) of an identified earliest activation point. In other examples, a further filter (or alternative filter) can include a distance (e.g., less than 20 millimeter, less than 10 millimeters, less than 5 millimeters, less than 1 millimeter) from the earliest activation point. In this way, a more precise map can be generated and displayed for the physician to identify locations that should be ablated. The time and distance from the earliest activation point can changed by the physician to identify locations for ablation depending on the type of arrhythmia or other abnormal condition identified.

As one non-limiting example, the disclosed technology can include the ability for a physician to set the map data to show any location where the activation time is within a predetermine time (e.g., less than one second, less than 10 milliseconds, less than 1 millisecond) and/or within a predetermined distance (e.g., less than 20 millimeter, less than 10 millimeters, less than 5 millimeters, less than 1 millimeter) to be illustrated in a first color (e.g., red) while all other locations are illustrated in a second color (e.g., purple).

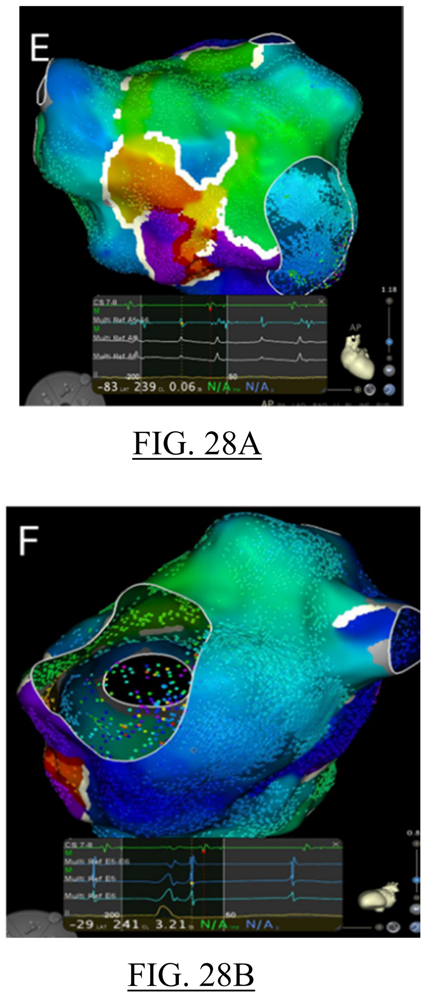

FIGs. 28A and 28B represent the intracardiac electrocardiograms recordings observed by the catheter of a patient's heart, in accordance with an example of the present invention. Filtering out far field ventricular signals during atrial tachycardia mapping and around lines of block, reducing incorrect annotations.

Acquired points were automatically annotated to the sharpest greatest negative deflection (-dV/dt) and delineating the earliest pre-P-wave intracardiac atrial signal with a QS complex and a sharp downstroke for focal ATs. Whereas the earliest PV breakthrough site was targeted to achieve vein isolation, 23/30 AT participants (76.7%) had one or more areas of interest, such as PV and non-PV triggers, PVI breakthrough, left atrial flutter critical isthmus, or CTI, identified by the investigational catheter.

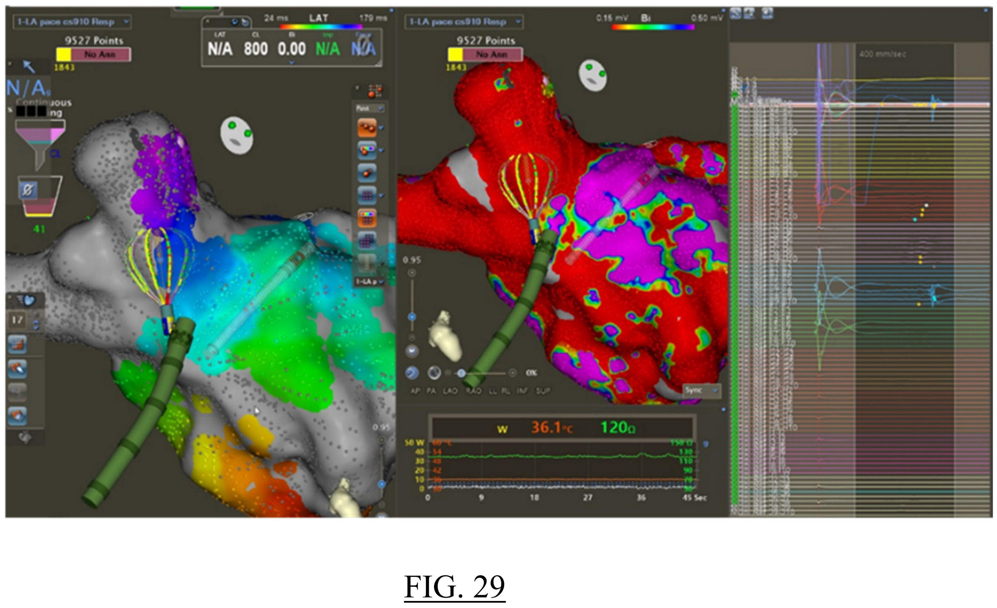

FIG. 29 illustrates a graphical display of the catheter positioned in a patient's heart to complete a mapping procedure, in accordance with an example of the present invention. As shown, pulmonary vein breakthrough was identified and mapped at the anterior right PV using the investigational catheter.

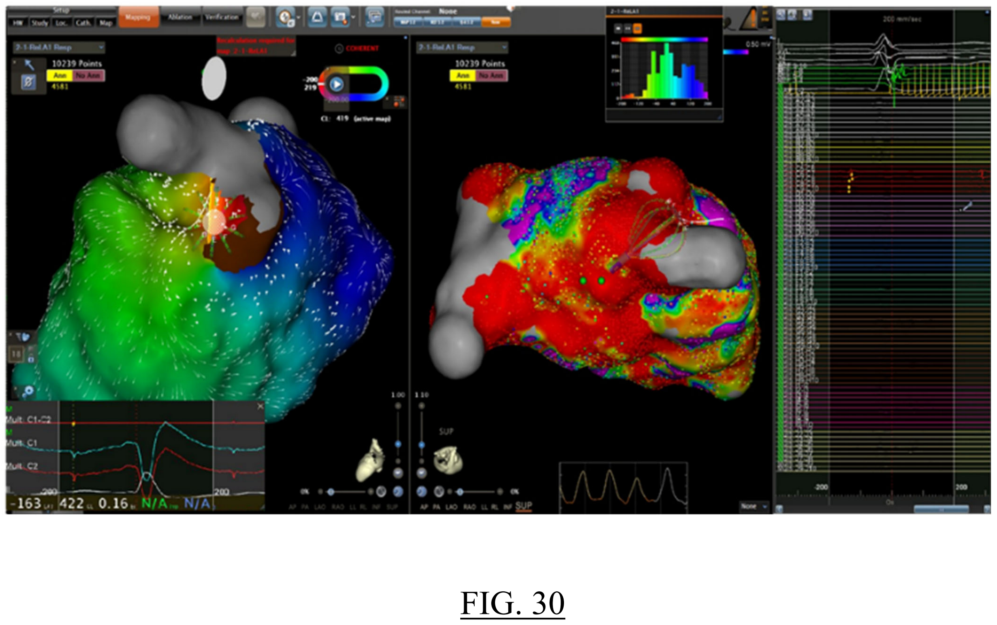

FIG. 30 illustrates a graphical display of the catheter positioned in a patient's heart showing the QS Pre-P wave focal atrial tachycardia site being mapped and identified at the anterior left carina area, in accordance with an example of the present invention. As shown, the earliest QS Pre-P- wave focal atrial tachycardia site was mapped and identified at the anterior left carina area.

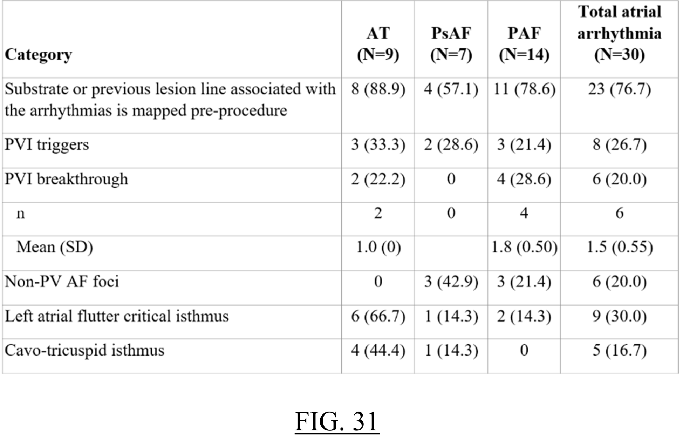

FIG. 31 is a table summarizing areas of interest identified using the catheter in participants with atrial tachycardia to include PVI triggers, PVI breakthroughs, non-PV AF Foci, left atrial critical isthmus, and Cavo tricuspid isthmus, in accordance with an example of the present invention.

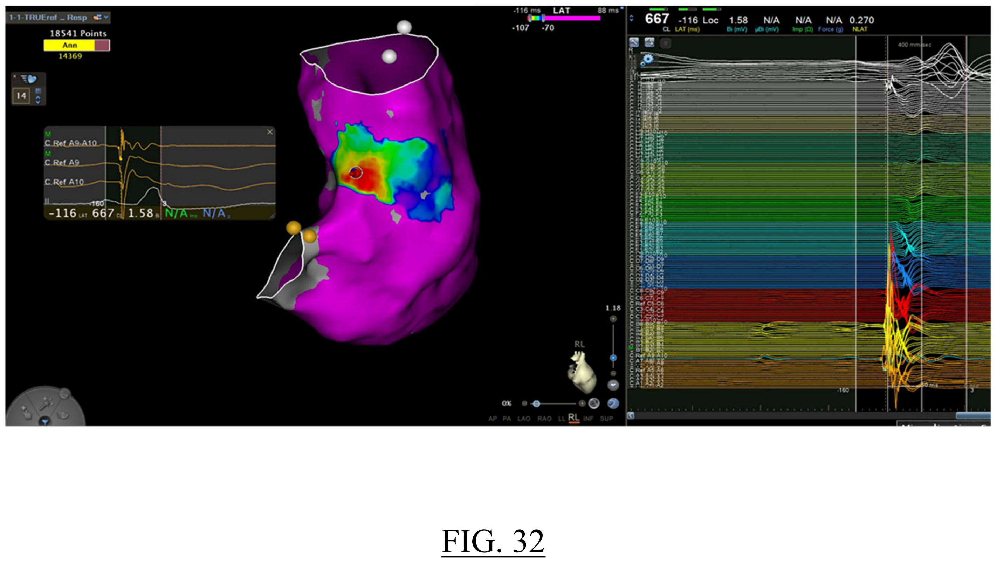

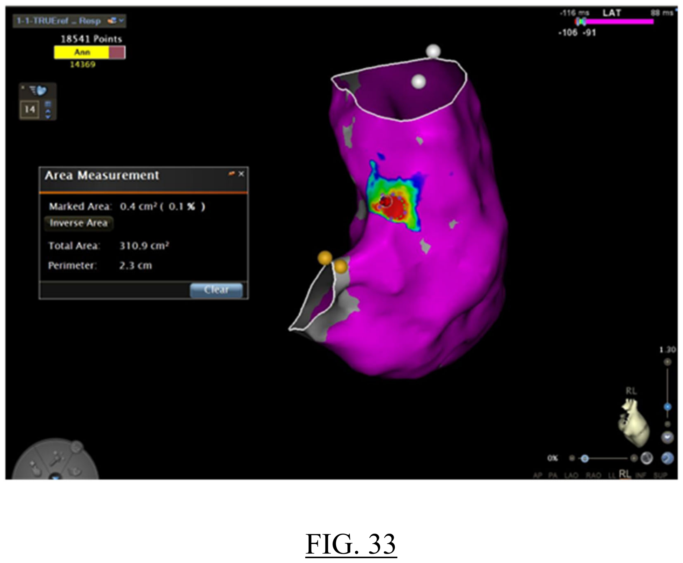

Furthermore, FIG. 32 illustrates a PVC map with the earliest QS pre-QRS site identified at the septal RVOT using the catheter and FIG. 33 illustrates an area and perimeter of the earliest activation timing locations less than a predetermined time duration calculated and compared to a map's reference, in accordance with an example of the present invention. Points were automatically annotated to the sharpest greatest negative deflection (-dV/dt) and delignating the earliest intracardiac pre-QRS ventricular signal with a QS complex and a sharp downstroke for PVCs. Since most of ventricular cases were PVCs and not ischemic VTs, it was not feasible to identify conduction channels, gaps, critical isthmuses and late potentials. However, the investigational catheter was able to identify and bracket the earliest points that activate within a predetermine time (e.g., 10 ms) to an average area of 1.08 cm² for all 9 PVC procedures.

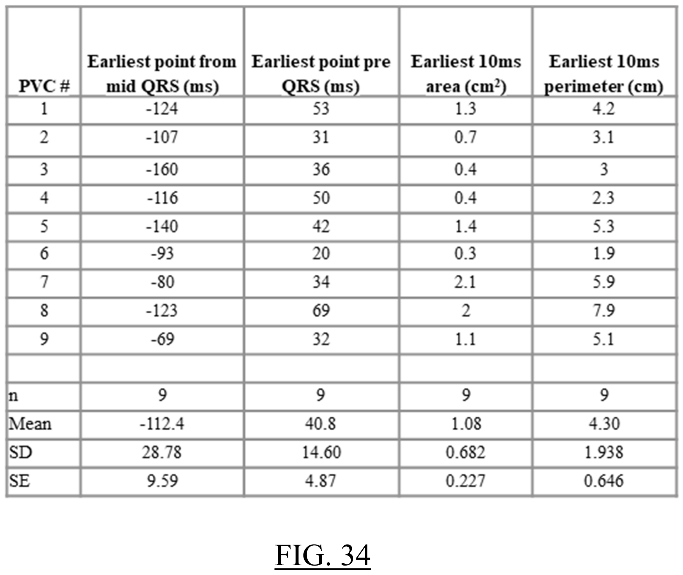

FIG. 34 is a table summarizing all PVC cases identifying earliest pot from mid QRS (reference), earliest point pre-QRS, area of the earliest predetermined time duration activation points (e.g., 10 milliseconds), and perimeter of the earliest 10 millisecond points, in accordance with an example of the present invention.

### Description of Method

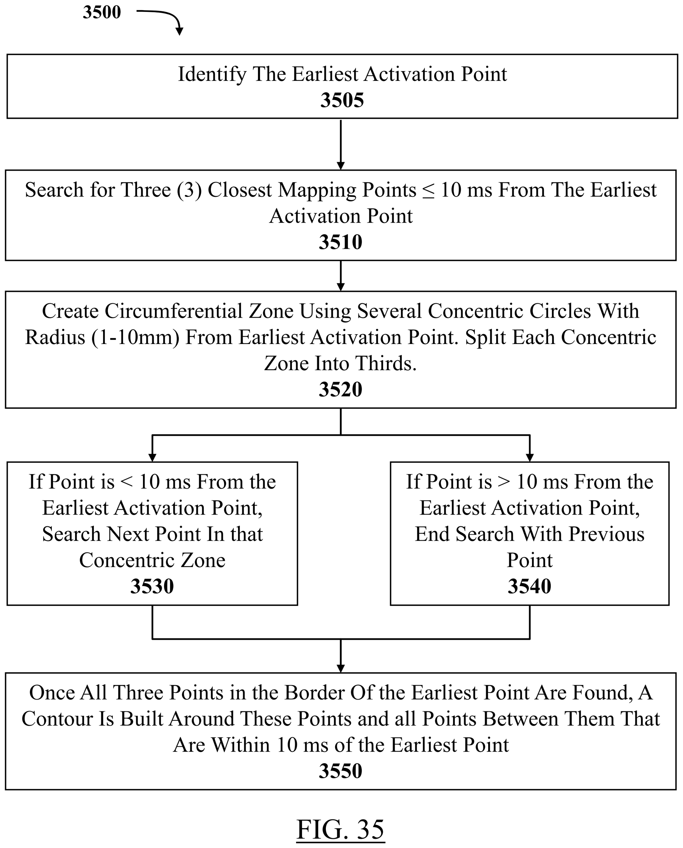

FIG. 35 illustrates a flow chart of a method 3500 of using the catheter, in accordance with an example of the present invention. As shown, the method 3500 can include identifying 3505 the earliest activation point based on the electrophysiological data collected by the catheter (catheter 10). Identifying 3505 the earliest activation point can be based on both the electrophysiological data collected by the electrodes 26 and the reference electrode 31 as well as position data collected by the position sensors 74. The method 3500 can further include searching 3510 for a plurality (e.g., three) closest mapping points that have activation times less than or equal to a predetermined time duration from the earliest activation time (e.g., less than one second, less than 10 milliseconds, less than 1 millisecond) and creating 3520 a circumferential zone using several concentric circles with a radius within a radius of a predetermine distance or distances (e.g., less than 20 millimeter, less than 10 millimeters, less than 5 millimeters, less than 1 millimeter) from the earliest activation point and splitting each concentric zone into thirds.

The method 3500 can further include, if a given point is less than the predetermined time duration from the earliest activation time at the earliest activation point, searching 3530 for a next point within the concentric zone. Alternatively, if a given point is greater than the predetermined time duration from the earliest activation time at the earliest activation point, ending 3540 the search with the previously-identified point.

Once all plurality of points in the border of the earliest point have been identified, the method 3500 can further include building 3550 a contour around the plurality of points and all points between them that are within the predetermine time from the earliest activation time at the earliest activation point. The method can further include outputting the result to a display so that the physician can observe the recorded data.

As will be appreciated, the method 3500 just described can help to more accurately identify locations of tissue that are to be ablated. Further, as previously described, the disclosed technology can include features that enable a physician to change settings depending on their preferred method and the type of arrhythmia identified so that the time and distance from the earliest activation point are changed to more accurately identify the location for ablation for the given scenario.

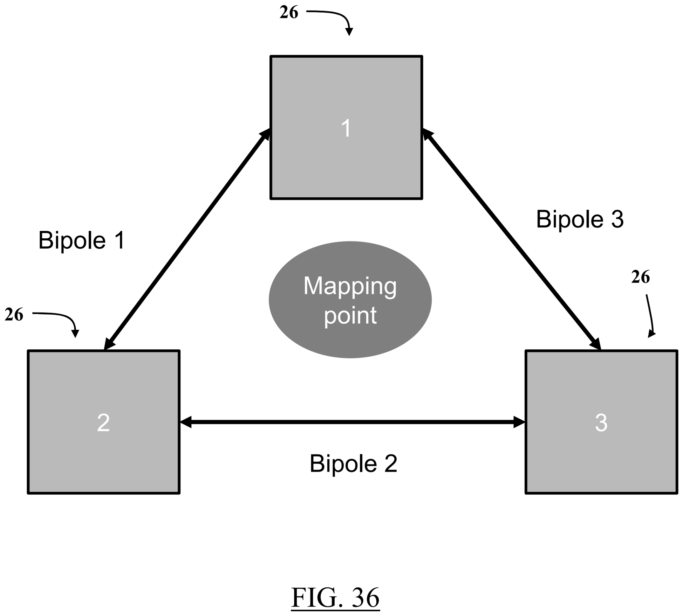

FIG. 36 illustrates a schematic of a method of using the catheter, in accordance with an example of the present invention. A mapping point can be identified by measuring electrophysiological signals a various electrodes 26 (in this example, three electrodes 26 are shown). Bipoles can be determined between each of the electrodes 26 and a mapping point can be identified between the plurality of electrodes 26. In this way, a more accurate representation of the electrophysiological data can obtained and displayed for a physician. For example, by using more than one electrode 26 and by setting up bipoles between a plurality of electrodes 26, inaccuracies due to direction wave propagation can be mitigated.

The disclosed technology described herein can be further understood according to the following clauses:
Clause 1: A method comprising: navigating a medical probe to a target location in a patient's heart, the medical probe extending along a longitudinal axis and comprising a plurality of spines configured to bow radially outward from the longitudinal axis, the plurality of spines comprising a plurality of electrodes disposed thereon and at least a location sensor disposed on the longitudinal axis, the location sensor configured to provide a location signal representative of a location of the sensor and the medical probe in the heart; receiving electrophysiological signals from at least some of the electrodes of the plurality of electrodes; identifying, based on the electrophysiological signals and the location signal, an earliest activation point identified as having the earliest activation time; identifying, based on the electrophysiological signals and the location signal, a plurality of points closest to the earliest activation point having an activation time less than a predetermined time duration from the earliest activation time; and generating an electro-anatomical map of the heart based on data corresponding to the plurality of points, the electro-anatomical map representing the location of the earliest activation point for subsequent ablation.
Clause 2: The method of clause 1 further comprising defining a circumferential zone using a plurality of concentric circles with a radius of less than 10 millimeters from the earliest activation point.
Clause 3: The method of clause 2 further comprising dividing each concentric circle into thirds.
Clause 4: The method of clause 2, wherein the radius is less than 7.5 millimeters from the earliest activation point.
Clause 5: The method of clause 2, wherein the radius is less than 5 millimeters from the earliest activation point.
Clause 6: The method of clause 2, wherein the radius is less than 2.5 millimeters from the earliest activation point.
Clause 7: The method of clause 2, wherein the radius is approximately 1 millimeter from the earliest activation point.
Clause 8: The method of any one of clauses 2-7 further comprising, if a concentric circle of the plurality of concentric circles comprises a point having an activation time of less than the predetermined time duration from the earliest activation time, identifying an additional point in the concentric circle having an activation time of less than the predetermined time duration from the earliest activation time.
Clause 9: The method of any one of clauses 2-7 further comprising, if a respective concentric circle of the plurality of concentric circles comprises a point having an activation time of greater than the predetermined time duration from the earliest activation time, using the previous point used to define the respective concentric circle.
Clause 10: The method of clauses 8 or 9, further comprising defining an area based on the plurality of points and any points between the plurality of points that comprise an activation time of less than the predetermined time duration from the earliest activation time.
Clause 11: The method of any one of the preceding clauses, wherein the plurality of points comprises at least three points.
Clause 12: The method of any one of the preceding clauses, wherein the plurality of points comprises exactly three points.
Clause 13: The method of any one of the preceding clauses, wherein the plurality of spines comprises ten spines and each spine of the plurality of spines comprises ten electrodes.
Clause 14: The method of any one of the preceding clauses, wherein the electrodes are disposed along a flexible printed circuit on respective spines of the plurality of spines.
Clause 15: The method of any one of the preceding clauses, wherein each electrode of the plurality of electrodes is coated with an impedance reducing coating.
Clause 16: The method of any one of the preceding clauses, wherein the medical probe further comprises an actuator configured to cause the spines to bow radially outward to define a basket having a diameter adjustable between approximately 3 millimeters to 18 millimeters.
Clause 17: The method of clause 16, wherein the electrodes are configured to receive electrophysiological data irrespective of the diameter size of the basket.
Clause 18: The method of any one of the preceding clauses, wherein generating the electro-anatomical map comprises generating a map having a resolution of at least 924 points per minute.
Clause 19: The method of clause 18, wherein the map has a resolution of approximately 1496 points per minute.
Clause 20: The method of any one of the preceding clauses, wherein the medical probe further comprises a reference electrode disposed in a cavity defined by the plurality of spines.
Clause 21: The method of clause 20, wherein the reference electrode is configured to receive electrophysiological data used to reduce far-field signal components.
Clause 22: The method of any one of the preceding clauses, wherein the at least one location sensor comprises a first magnetic sensor disposed at a distal end of the plurality of spines and a second magnetic sensor disposed at a proximal end of the plurality of spines.
Clause 23: The method of any one of the preceding clauses, wherein the medical probe further comprises one or more position sensing electrodes disposed on a shaft of the medical probe, the one or more position sensing electrodes being configured for impedance based position sensing.
Clause 24: The method of any one of the preceding clauses, wherein a primary safety endpoint of the method comprises no occurrence of one or more serious adverse events within seven days from generating the electro-anatomical map, the serious adverse events comprising: death, a life-threatening illness or injury, permanent impairment of a body structure or function, in-patient hospitalization or prolongation of an existing hospitalization, medical or surgical intervention to prevent life-threatening illness or injury or permanent impairment to body structure or function, chronic disease, or an event that leads to fetal distress, fetal death or a congenital abnormality or birth defect.
Clause 25: The method of any of the preceding clauses, wherein the predetermined time duration comprises less than or equal to one second.
Clause 26: The method of any of clause 1-24, wherein the predetermined time duration comprises less than or equal to 10 milliseconds.
Clause 27: The method of any of clauses 1-24, wherein the predetermined time duration comprises less than or equal to 5 milliseconds.
Clause 28: The method of any of clauses 1-24, wherein the predetermined time duration comprises less than or equal to 1 millisecond.
Clause 29: A medical system comprising: a medical probe comprising: a shaft extending along a longitudinal axis; a plurality of spines disposed at a distal end of the shaft and configured to bow radially outward from the longitudinal axis to define a cavity therebetween; a location sensor disposed on the longitudinal axis, the location sensor configured to provide a location signal representative of a location of the sensor and the medical probe in a heart; a plurality of electrodes disposed along the plurality of spines; and a reference electrode disposed in the cavity; one or more processors; and a memory storing instructions that, when executed by the one or more processors, are configured to cause the medical system to: receive electrophysiological signals from at least some of the electrodes of the plurality of electrodes; identify, based on the electrophysiological signals and the location signal, an earliest activation point identified as having the earliest activation time; identify, based on the electrophysiological signals and the location signal, a plurality of points closest to the earliest activation point having an activation time less than the predetermined time duration from the earliest activation time; and generate an electro-anatomical map of the heart based on data corresponding to the plurality of points, the electro-anatomical map representing the location of the earliest activation point for subsequent ablation.
Clause 30: The medical system of clause 29 the instructions, when executed by the one or more processors, are further configured to cause the medical system to define a circumferential zone using a plurality of concentric circles with a radius of less than 10 millimeters from the earliest activation point.
Clause 31: The medical system of clause 30 wherein the instructions, when executed by the one or more processors, are further configured to divide each concentric circle into thirds.
Clause 32: The medical system of clause 30, wherein the radius is less than 7.5 millimeters from the earliest activation point.
Clause 33: The medical system of clause 30, wherein the radius is less than 5 millimeters from the earliest activation point.
Clause 34: The medical system of clause 30, wherein the radius is less than 2.5 millimeters from the earliest activation point.
Clause 35: The medical system of clause 30, wherein the radius is approximately 1 millimeter from the earliest activation point.
Clause 36: The medical system of any one of clauses 30-35, wherein the instructions, when executed by the one or more processors, are further configured to cause the medical system to: if a concentric circle of the plurality of concentric circles comprises a point having an activation time of less than the predetermined time duration from the earliest activation time, identify an additional point in the concentric circle having an activation time of less than 10 milliseconds from the earliest activation time.
Clause 37: The medical system of any one of clauses 30-36, wherein the instructions, when executed by the one or more processors, are further configured to cause the medical system to, if a respective concentric circle of the plurality of concentric circles comprises a point having an activation time of greater than the predetermined time duration from the earliest activation time, use the previous point used to define the respective concentric circle.
Clause 38: The medical system of clauses 36 or 37, wherein the instructions, when executed by the one or more processors, are further configured to cause the medical system to define an area based on the plurality of points and any points between the plurality of points that comprise an activation time of less than the predetermined time duration from the earliest activation time.
Clause 39: The medical system of any one of clauses 29-38, wherein the plurality of points comprises at least three points.
Clause 40: The medical system of any one of clauses 29-39, wherein the plurality of points comprises exactly three points.
Clause 41: The medical system of any one of clauses 29-40, wherein the plurality of spines comprises ten spines and each spine of the plurality of spines comprises ten electrodes.
Clause 42:_The medical system of any one of clauses 29-41, wherein the electrodes are disposed along a flexible printed circuit on respective spines of the plurality of spines.
Clause 43: The medical system of any one of clauses 29-42, wherein each electrode of the plurality of electrodes is coated with an impedance reducing coating.
Clause 44: The medical system of any one of clauses 29-43, wherein the medical probe further comprises an actuator configured to cause the spines to bow radially outward to define a basket having a diameter adjustable between approximately 3 millimeters to 18 millimeters.
Clause 45: The medical system of clause 44, wherein the electrodes are configured to receive electrophysiological data irrespective of the diameter size of the basket.
Clause 46: The medical system of any one of clauses 29-45, wherein generating the electro-anatomical map comprises generating a map having a resolution of at least 924 points per minute.
Clause 47: The medical system of clause 46, wherein the map has a resolution of approximately 1496 points per minute.
Clause 48: The medical system of any one of clauses 29-47, wherein the medical probe further comprises a reference electrode disposed in a cavity defined by the plurality of spines.
Clause 49: The medical system of clause 48, wherein the reference electrode is configured to receive electrophysiological data used to reduce far-field signal components.
Clause 50: The medical system of any one of clauses 29-49, wherein the medical probe further comprises a first magnetic sensor disposed at a distal end of the plurality of spines and a second magnetic sensor disposed at a proximal end of the plurality of spines.
Clause 51: The medical system of any one of clauses 29-50, wherein the medical probe further comprises one or more electrodes disposed on the shaft configured for impedance based position sensing.
Clause 52: The medical system of any of clauses 29-51, wherein the predetermined time duration comprises less than or equal to one second.
Clause 53: The medical system of any of clauses 29-51, wherein the predetermined time duration comprises less than or equal to 10 milliseconds.
Clause 54: The medical system of any of clauses 29-51, wherein the predetermined time duration comprises less than or equal to 5 milliseconds.
Clause 55: The medical system of any of clauses 29-51, wherein the predetermined time duration comprises less than or equal to 1 millisecond.

The embodiments described above are cited by way of example, and the present invention is not limited by what has been particularly shown and described hereinabove. Rather, the scope of the invention includes both combinations and sub combinations of the various features described and illustrated hereinabove, as well as variations and modifications thereof which would occur to persons skilled in the art upon reading the foregoing description and which are not disclosed in the prior art.

## Claims

1. A medical system comprising:
a medical probe comprising:
a shaft extending along a longitudinal axis;
a plurality of spines disposed at a distal end of the shaft and configured to bow radially outward from the longitudinal axis to define a cavity therebetween;
a location sensor disposed on the longitudinal axis, the location sensor configured to provide a location signal representative of a location of the sensor and the medical probe in a heart;
a plurality of electrodes disposed along the plurality of spines; and
a reference electrode disposed in the cavity;
one or more processors; and
a memory storing instructions that, when executed by the one or more processors, are configured to cause the medical system to:
receive electrophysiological signals from at least some of the electrodes of the plurality of electrodes;
identify, based on the electrophysiological signals and the location signal, an earliest activation point identified as having the earliest activation time;
identify, based on the electrophysiological signals and the location signal, a plurality of points closest to the earliest activation point having an activation time less than a predetermined time duration from the earliest activation time; and
generate an electro-anatomical map of the heart based on data corresponding to the plurality of points, the electro-anatomical map representing the location of the earliest activation point for subsequent ablation.

2. The medical system of claim 1, wherein the instructions, when executed by the one or more processors, are further configured to cause the medical system to define a circumferential zone using a plurality of concentric circles with a radius of less than 10 millimeters from the earliest activation point.

3. The medical system of claim 2 wherein the instructions, when executed by the one or more processors, are further configured to divide each concentric circle into thirds.

4. The medical system of claim 2 or claim 3, wherein the instructions, when executed by the one or more processors, are further configured to cause the medical system to: if a concentric circle of the plurality of concentric circles comprises a point having an activation time of less than the predetermined time duration from the earliest activation time, identify an additional point in the concentric circle having an activation time of less than the predetermined time duration from the earliest activation time.

5. The medical system of claim 2 or claim 3, wherein the instructions, when executed by the one or more processors, are further configured to cause the medical system to, if a respective concentric circle of the plurality of concentric circles comprises a point having an activation time of greater than the predetermined time duration from the earliest activation time, use the previous point used to define the respective concentric circle.

6. The medical system of claim 4 or claim 5, wherein the instructions, when executed by the one or more processors, are further configured to cause the medical system to define an area based on the plurality of points and any points between the plurality of points that comprise an activation time of less than the predetermined time duration from the earliest activation time.

7. The medical system of any preceding claim, wherein the plurality of points comprises at least three points.

8. The medical system of any preceding claim, wherein the plurality of spines comprises ten spines and each spine of the plurality of spines comprises ten electrodes.

9. The medical system of any preceding claim, wherein the electrodes are disposed along a flexible printed circuit on respective spines of the plurality of spines.

10. The medical system of any preceding claim, wherein each electrode of the plurality of electrodes is coated with an impedance reducing coating.

11. The medical system of any preceding claim, wherein the medical probe further comprises an actuator configured to cause the spines to bow radially outward to define a basket having a diameter adjustable between approximately 3 millimeters to 18 millimeters.

12. The medical system of any preceding claim, wherein generating the electro-anatomical map comprises generating a map having a resolution of at least 924 points per minute.

13. The medical system of any preceding claim, wherein the medical probe further comprises a reference electrode disposed in a cavity defined by the plurality of spines, optionally wherein the reference electrode is configured to receive electrophysiological data used to reduce far-field signal components.

14. The medical system of any preceding claim, wherein the medical probe further comprises (i) a first magnetic sensor disposed at a distal end of the plurality of spines and a second magnetic sensor disposed at a proximal end of the plurality of spines, and/or (ii) one or more electrodes disposed on the shaft configured for impedance based position sensing.

15. The medical system of any preceding claim, wherein the predetermined time duration comprises less than or equal to 10 milliseconds.
